# EUROPEAN PATENT APPLICATION

(11) **EP 4 257 133 A1**
(43) Date of publication of application: **11.10.2023**
(21) Application number: 22305461.0
(22) Date of filing: 05.04.2022
(51) Int. Cl.: A61K 31/517, A61K 31/506, A61K 31/513, A61K 31/519, A61K 31/5513, A61P 33/06, A61K 45/06

(54) **OXO-AZAHETEROCYCLIC DERIVATIVES FOR USE IN THE TREATMENT OF MALARIA**

(71) Applicant: Institut Pasteur, 75015 Paris (FR)
(72) Inventor: CHITNIS, Chaitanya, 75015 Paris (FR); WAGNER, Matthias, 75015 Paris (FR); SPERANDIO, Olivier, 75015 Paris (FR); CHECA RUANO, Luis, 75015 Paris (FR); TOUQUI, Lhousseine, 75015 Paris (FR); GORGETTE, Olivier, 75015 Paris (FR); HUON, Christèle, 75015 Paris (FR); FORMAGLIO, Pauline, 75015 Paris (FR); AMINO, Rogerio, 75015 Paris (FR); ALAGANAN, Aditi, 75015 Paris (FR)
(74) Representative: Gevers & Orès

(57) **Abstract**

The present invention is in the field of therapeutic drugs to treat malaria. In particular, the invention provides oxo-azaheterocyclic derivatives for use in the treatment of malaria, for example drug-resistant malaria.

## Description

The present invention is in the field of therapeutic drugs to treat malaria. In particular, the invention provides oxo-azaheterocyclic derivatives for use in the treatment of malaria, for example drug-resistant malaria.

Malaria is a devastating infectious disease which affect each year more than 229 million people and claimed 409,000 deaths worldwide in 2019. As a major health concern in Africa, Asia, the Middle East, and Central and South America, about 40% of the world's population live in areas where malaria is transmitted. Approximately 90% of both cases and deaths occurred in Africa, mostly among children, 78% of reported deaths belonging to children below 5 years age.

Malaria thus remains a major public health problem in the tropical world.

Malaria is caused by blood protozoa of the genus Plasmodium, transmitted by Anopheles spp., and shows symptoms such as intermittent paroxysm of fever, anemia, splenomegaly, etc. The five known species of *Plasmodium genus* that causes malaria in human are *Plasmodium falciparum, Plasmodium vivax, Plasmodium malariae, Plasmodium ovale,* and *Plasmodium knowlesi.*

Among them, *P. falciparum* is the most virulent parasite, with high mortality and morbidity rate, causing the most severe symptoms: it progresses to severe malaria easily at 1 or 2 days after the onset of disease, accompanied with coma and multiple organ failure that eventually leads to the death of the patients.

All clinical symptoms of malaria are attributed to the blood stage of the parasite's life cycle where *P. falciparum* invades and multiplies within host red blood cells (RBCs).

The discovery of the synthetic drug chloroquine (CQ, first of the quinoline class) in the 1940s helped to treat and prevent malaria throughout the world in 1950s. However, the therapeutic efficacy of CQ and efforts to eradicate malaria worldwide were diminished due to the occurrence of CQ resistance. The failure of these eradication programs led to re-emergence of malaria and spread of CQ-resistant parasite from Southeast Asia and South America to Africa. Due to the lack of potent and affordable drug for malaria treatment, the spread of CQ-resistant parasite to Africa around 1980s claimed 2-3-fold increase in malaria-related deaths. Hence, CQ was replaced with sulfadoxine/pyrimethamine (SP, of the antifolate class) as the first-line of treatment for malaria; however, parasite became resistant to SP and spread widely. Resistance to the current state-of-the-art antimalarial drug artemisinin (ART) in *P. falciparum,* which was first reported in Southeast Asia in the early 2000s, has now been detected in sub-Saharan Africa.

Mechanistically, *P. falciparum* attains artemisinin resistance by reducing the uptake of host red blood cell hemoglobin, which in turn leads to lower oxidative stress levels and prevents activation of artemisinin, enabling parasite survival.

During blood stage growth, *P. falciparum* internalizes hemoglobin in an endocytic process known as host cell cytosol uptake (HCCU). Endocytosed hemoglobin is transported in vesicles to an acidic, lysosome-like parasite organelle termed food vacuole (FV), where hemoglobin is proteolytically digested to provide amino acids for synthesis of parasite proteins. The digestion of hemoglobin releases free heme which is partly detoxified by crystallization into hemozoin. The Fe(II) ion within the free heme catalyses the production of reactive oxygen species (ROS) that cause the oxidation of unsaturated membrane phospholipids (PLs). This process termed lipid oxidation is toxic for cells as it causes drastic changes to membrane fluidity, rigidity, curvature and permeability.

Use of antimalarial drugs as combination therapy instead of monotherapy has been in practice to increase the efficacy of drug and to delay the emergence of drug resistance parasite. Since 2001, artemisinin-based combination therapies (ACTs) has been most widely and effectively used for malaria treatment. Recently, resistance to ACTs (Artesunate-Mefloquine and Dihydroartemisinin-Piperaquine) has been reported in Southeast Asia (Greater Mekong Subregion, GMS) increasing the global alarm for malaria treatment and control.

The risk of ACT-resistant parasites spreading from the Greater Mekong Subregion to Africa is extremely worrisome, as it happened previously with chloroquine- and sulfadoxine/pyrimethamine-resistant parasites.

Drug resistant malaria is thus a serious clinical and public health problem, which poses a major threat to malaria control and elimination efforts. Therefore, novel pharmaceutical compositions which kill drug-resistant malaria parasites are needed for successful therapy.

The present invention thus concerns a compound of following formula (I): 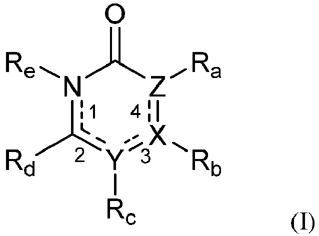 wherein:
- X is C, Y is C and Z is N, bonds 1 and 3 being double bonds, bonds 2 and 4 being single bonds; or
- X is C, Y is N and Z is C, bond 3 is a single bond and bond 4 is a double bond, and:
   ∘ bond 1 is a double bond, bond 2 is a single bond, Rₑ being absent; or
   ∘ bond 1 is a singe bond, bond 2 is a double bond, R_{c} being absent; or
- X is N, Y is C, Z is C, bonds 1 and 3 being single bonds, bonds 2 and 4 being double bonds; or
- X and Z are C and Y-R_{c} is 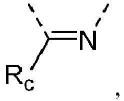 bonds 1, 2 and 3 are single bonds, bond 4 is a double bond;
   when Z is C, Ra is hydrogen, halogen, (C₁-C₃)-alkyl, (C₁-C₃)-alkoxy, hydroxyl-(C₁-C₃)-alkyl, (C₁-C3)-alkylthio, (C₁-C₃)-alkylsulphinyl, amino-(C₁-C₃)-alkyl, mono- or di-(C₁-C₃)-alkylamino-(C₁-C3)-alkyl, (C₁-C₃)-alkylcarbonylamino-(C₁-C₃)-alkyl, (C₁-C₃)-alkoxy-(C₁-C₃)-alkylcarbonylamino-(C₁-C₃)-alkyl, (C₁-C₃)-alkylsulphonylamino-(C₁-C₃)-alkyl, (C₁-C₃)-alkylcarboxy, (C₁-C₃)-alkylcarboxy(C₁-C₃)-alkyl, aryl, in particular phenyl, or aralkyl, in particular benzyl, said aryl being optionally substituted by 1, 2, 3 or 4 substituents which may be the same or different selected from halogen, (C₁-C₄)-alkyl, CN, CF3, OCF3, COR2, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio, or mono to perfluoro-(C₁-C₄)-alkyl);
   when Z is N, Ra is hydrogen, NR₄R₅, (C₁-C₆)-alkyl, (C₁-C₄)-alkoxy-(C₁-C₆)-alkyl, aryl, aryl-(C₁-C6)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C6)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
   R_{b} is hydrogen, halogen, (C₁-C₃)-alkyl, hydroxyl-(C₁-C₃)-alkyl, NR₄R₅, in particular NH2, OH or (C₁-C₃)-alkoxy, with the proviso that Ra and R_{b} are not simultaneously each hydrogen; or when Y is N, Ra and R_{b} together may be (CH₂)ₙ where n is 3 or 4, to form, with the main ring carbon atoms to which they are attached a fused 5-or 6-membered carbocyclic ring; or when Y is N, Ra and R_{b} together with the main ring carbon atoms to which they are attached may form a fused benzo or heteroaryl ring optionally substituted by 1, 2, 3 or 4 substituents which may be the same or different selected from halogen, (C₁-C₄)-alkyl, CN, CF3, OCF3, COR2, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio, or mono to perfluoro-(C₁-C₄)-alkyl); or
   when Y is N, Ra and R_{b} together with the main ring carbon atoms to which they are attached may form a fused heterocycle, in particular a fused azaheterocycle, optionally substituted by 1, 2, 3 or 4 substituents which may be the same or different selected from (C₁-C₄)-alkyl, oxo, aryl or heteroaryl ring which is further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy), (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
   said fused azaheterocycle optionally comprises at least one N-R_{a'} member;
   R_{a'} is (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
   R₂ and R₃ are independently hydrogen or (C₁-C₂₀)-alkyl, for instance (C₁-C₄)-alkyl;
   R_{2'} is R₂ or NR₄R₅;
   R_{3'} is (C₁-C₁₂)-alkyl, preferably (C₁-C₆)-alkyl, an aryl or heteroaryl group, said group being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl;
   R₄ is hydrogen, (C₁-C₁₂)-alkyl, preferably (C₁-C₆)-alkyl, (C₁-C₁₂)-alkyl-R_{4'}, preferably (C₁-C₆)-alkyl-R_{4'};
   R_{4'} is NH₂, mono- or di-(C₁-C₃)-alkylamino;
   R₅ is hydrogen, (C₁-C₁₂)-alkyl, preferably (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl group being optionally fused with a cycloalkane, in particular a (C₅-C₈)-cycloalcane or a 5- to 8- membered heterocycle comprising N and optionally O or S, and being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl; or
   R₄ and R₅ together with the nitrogen to which they are attached form a 5- to 8 membered ring optionally containing one or more further heteroatoms selected from oxygen, nitrogen, notably as N-R_{a'} member, and sulphur, optionnaly fused with an aromatic ring, preferably a benzene ring, said rings being optionally substituted by one or two substituents selected from hydroxy, oxo, (C₁-C4)-alkyl, (C₁-C₄)-alkyl-CO, aryl, in particular phenyl, or aralkyl, in particular benzyl, for example morpholine or piperazine; or
   R₄ and R₅ which may be the same or different is each selected from CH₂R₆, CHR₇CO₂H or a salt thereof in which:
      R₆ is COOH or a salt thereof, COOR₈, CONR₂R₃, CN, CH₂OH or CH₂OR₂;
      R₇ is an amino acid side chain such as CH₂OH from serine;
      R₈ is (C₁-C₄)-alkyl or a pharmaceutically acceptable *in vivo* hydrolysable ester group;
      When Y is N, R_{c} is (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy; or
      When Y is N, R_{c} is of following formula (A): 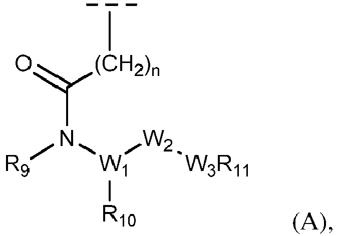
      R₉ is hydrogen, (C₁-C₆)-alkyl which may be unsubstituted or substituted by 1, 2 or 3 substituents selected from hydroxy, halogen, OR2, COR2, carboxy, COOR2, CONR₄R₅, NR₄R₅, NR₄COR₅, mono-or di-(hydroxyl-(C₁-C₆)-alkyl)amino and N-hydroxy-(C₁-C₆)-alkyl-N-(C₁-C₆)-alkylamino, for example 1-piperidinoethyl; or
      R₉ is Het-(C₀-C₄)-alkyl in which Het is a 5- to 7- membered heterocyclyl ring comprising N and optionally O or S, bonded through a carbon ring atom and in which N may be substituted by COR₂, COOR2, CONR₄R₅, or (C₁-C₆)-alkyl optionally substituted by 1, 2 or 3 substituents selected from hydroxy, halogen, OR2, COR2, carboxy, COOR2, CONR₄R₅, NR₄R₅, NR₄COR₅, for instance, piperidin-4-yl, pyrrolidin-3-yl;
      R₁₀ is hydrogen or (C₁-C₃)-alkyl;
      R11 is an aryl or heteroaryl ring which is further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy), (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
      W₁ is (CH₂)ₚ(O)_{q} in which p is 1, 2 or 3 and q is 0 or p is 2 or 3 and q is 1;
      W2 is an arene diyl or heteroarene diyl group which is further optionally substituted by 1, 2, 3 or 4 substituents which may be the same or different selected from (C₁-C₁₈)-alkyl, in particular (C₁-C6)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl and mono to perfluoro-(C₁-C₄)-alkoxy;
      W₃ is O or a bond;
      when Y is C, R_{c} is H or in particular (C₁-C₆)-alkyl; or
      when X and Y are C, R_{c} and R_{b} together may be (CH₂)ₙ where n is 3 or 4, to form, with the main ring carbon atoms to which they are attached a fused 5-or 6-membered carbocyclic ring;
      when Y-R_{c} is 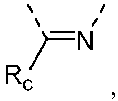 R_{c} is OH or (C₁-C₁₈)-alkoxy, in particular (C₁-C₆)-alkoxy;
      R_{d} is H or (C₁-C₆)-alkyl; or
      Rd is of the following formula (B): 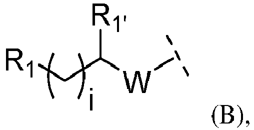
      i is 0 or 1;
      W is S, O, or CH2, in particular S;
      R₁ is COOR2, COR_{2'}, OR_{3'}, an aryl or heteroaryl group, said group being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C4)-alkyl, mono to perfluoro-(C₁-C₄)-alkoxyaryl, and aryl-(C₁-C₄)-alkyl;
      R_{1'} is H or (C₁-C₃)-alkyl;
      when X is N, R_{c} and R_{d} may together with the main ring carbon atoms to which they are attached form a fused benzo or heteroaryl ring optionally substituted by 1, 2, 3 or 4 substituents which may be the same or different selected from halogen, (C₁-C₄)-alkyl, CN, CF3, OCF3, COR2, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio, or mono to perfluoro-(C₁-C₄)-alkyl);
      Rₑ is H, (C₁-C₆)-alkyl, (C₁-C₆)-alkyl-R_{e'}, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
      R_{e'} is COR2, carboxy, COOR2, CONR₄R₅;
      with the proviso that:
- R_{d} is of formula (B); and/or
- Y is N, and R_{c} is aryl, aryl-(C₁-C₆)-alkyl, heteroaryl, heteroaryl-(C₁-C₆)-alkyl, or of formula (A); and/or
- Rₑ is (C₁-C₆)-alkyl-R_{e'}, aryl, aryl-(C₁-C₆)-alkyl, heteroaryl or heteroaryl-(C₁-C₆)-alkyl; or a pharmaceutically acceptable salt or solvate thereof,
for use in the treatment and/or prevention of malaria.

The inventors have shown that *P. falciparum* internalizes host RBC PRDX6 along with hemoglobin during blood stage parasite development (Figure 1). They further demonstrated that inhibition of human PRDX6 with the PLA2 inhibitor Darapladib blocks blood stage parasite development and growth, identifying PRDX6 as a novel host-derived drug target against malaria parasites (Figure 2). The inventors confirmed that compounds of the invention inhibit the PLA2 activity of PRDX6 (Figure 3). Moreover, co-treatment with a compound of the invention and artemisinin synergistically reduces survival of ART-resistant *P. falciparum* strains in a ring stage survival assay with artemisinin (Figure 4).

Compounds of the invention thus enable progress towards malaria elimination, through a novel strategy for pharmacological treatment and control of malaria.

Compounds of the invention also provide a valuable strategy to overcome artemisinin resistance in *P. falciparum.* In addition, since the compounds of the invention target host enzymes, the approach of the present invention is particularly attractive because parasites cannot mutate the target gene to attain resistance. Thus, the compounds of the invention are less likely to face the problem of drug resistance and may play a critical role in malaria eradication efforts in the long term.

In a particular embodiment, the compound for use as defined above is of formula (I) with the proviso that:
- Rd is of formula (B), with R₁ being an optionally substituted aryl or heteroaryl group, or being substituted by an aryl or heteroaryl group; and/or
- Y is N, and R_{c} is aryl, aryl-(C₁-C₆)-alkyl, heteroaryl, heteroaryl-(C₁-C₆)-alkyl, or of formula (A); and/or
- Rₑ is (C₁-C₆)-alkyl-R_{e'}, aryl, aryl-(C₁-C₆)-alkyl, heteroaryl or heteroaryl-(C₁-C₆)-alkyl, Rₑ being in particular aryl, aryl-(C₁-C₆)-alkyl, heteroaryl or heteroaryl-(C₁-C₆)-alkyl; and/or
- Rₐ and R_{b} together with the main ring carbon atoms to which they are attached form an optionally substituted fused benzo or heteroaryl ring; and/or
- R_{c} and R_{d} together with the main ring carbon atoms to which they are attached form an optionally substituted fused benzo or heteroaryl ring.

In a particular embodiment, the compound for use as defined above is of following formula (II): 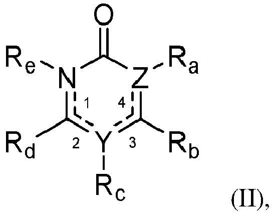 in particular of following formula (IIa):

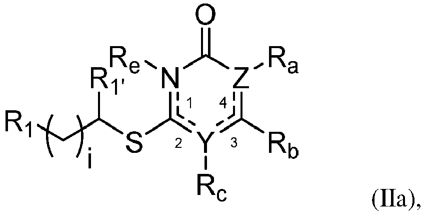

Wherein :
- Y is C and Z is N, bonds 1 and 3 being double bonds, bonds 2 and 4 being single bonds; or
- Y is N and Z is C, bond 3 is a single bond and bond 4 is a double bond, and:
   ∘ bond 1 is a double bond, bond 2 is a single bond, Rₑ being absent; or
   ∘ bond 1 is a singe bond, bond 2 is a double bond, R_{c} being absent.

In a particular embodiment, the compound for use as defined above is of following formula (III): 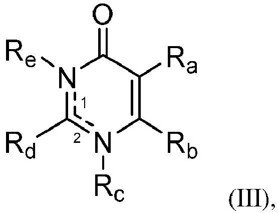 in particular of following formula (IIIa):

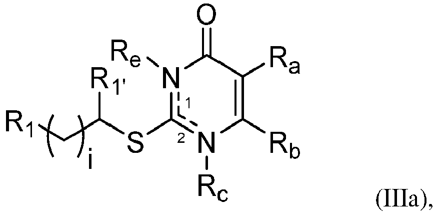

Wherein :
bond 1 and bond 2 are a single or a double bond, with:
- bond 1 is a double bond, bond 2 is a single bond, Rₑ being absent; or
- bond 1 is a singe bond, bond 2 is a double bond, R_{c} being absent.

In a particular embodiment, the compound for use as defined above is of following formula (IV):

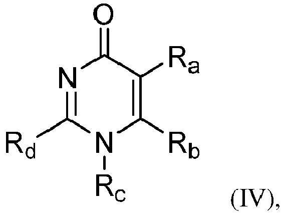

Wherein Ra, Rb, R_{c} and Rd are as defined above, and in particular wherein:
Ra is aryl, in particular phenyl, or aralkyl, in particular benzyl, said aryl being optionally substituted by 1, 2, 3 or 4 substituents which may be the same or different selected from halogen, (C₁-C₄)-alkyl, CN, CF3, OCF3, COR2, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio, or mono to perfluoro-(C₁-C₄)-alkyl), said aryl being more particularly phenyl;
R_{b} is hydrogen or (C₁-C₃)-alkyl, in particular hydrogen ;
said fused azaheterocycle optionally comprises at least one N-R_{a'} member;
R_{a'} is (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
R₂ and R₃ are independently hydrogen or (C₁-C₂₀)-alkyl, for instance (C₁-C₄)-alkyl;
R_{2'} is R₂ or NR₄R₅;
R_{3'} is (C₁-C₁₂)-alkyl, preferably (C₁-C₆)-alkyl, an aryl or heteroaryl group, said group being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl;
R₄ is hydrogen, (C₁-C₁₂)-alkyl, preferably (C₁-C₆)-alkyl, (C₁-C₁₂)-alkyl-R_{4'}, preferably (C₁-C₆)-alkyl-R_{4'};
R_{4'} is NH₂, mono- or di-(C₁-C₃)-alkylamino;
R₅ is hydrogen, (C₁-C₁₂)-alkyl, preferably (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl group being optionally fused with a cycloalkane, in particular a (C₅-C₈)-cycloalcane or a 5- to 8- membered heterocycle comprising N and optionally O or S, and being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl; or
R₄ and R₅ together with the nitrogen to which they are attached form a 5- to 8 membered ring optionally containing one or more further heteroatoms selected from oxygen, nitrogen, notably as N-R_{a'} member, and sulphur, optionnaly fused with an aromatic ring, preferably a benzene ring, said rings being optionally substituted by one or two substituents selected from hydroxy, oxo, (C₁-C4)-alkyl, (C₁-C₄)-alkyl-CO, aryl, in particular phenyl, or aralkyl, in particular benzyl, for example morpholine or piperazine;
R_{c} is aryl-(C₁-C₆)-alkyl, in particular an aryl-(C₁-C₂)-alkyl, or heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy, more particularly from (C₁-C₆)-alkyl, aryl-(C₁-C₆)-alkoxy and OCF₃;
R_{d} is H or (C₁-C₆)-alkyl.

In a particular embodiment, the compound for use as defined above is of following formula (IV): 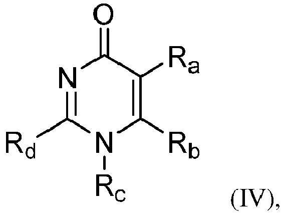 wherein:
Ra is aryl, in particular phenyl, or aralkyl, in particular benzyl, said aryl being optionally substituted by 1, 2, 3 or 4 substituents which may be the same or different selected from halogen, (C₁-C₄)-alkyl, CN, CF3, OCF3, COR2, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio, or mono to perfluoro-(C₁-C₄)-alkyl), said aryl being more particularly phenyl;
R_{b} is hydrogen or (C₁-C₃)-alkyl, in particular hydrogen or Me, more particularly Me ;
R_{c} is aryl-(C₁-C₆)-alkyl, in particular an aryl-(C₁-C₂)-alkyl, or heteroaryl-(C₁-C₆)-alkyl, in particular a heteroaryl-(C₁-C₂)-alkyl, said heteroaryl being for example indolyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy, more particularly from (C₁-C₆)-alkyl, aryl-(C₁-C₆)-alkoxy and OCF₃;
Rd is H or (C₁-C₆)-alkyl, more particularly Me.

In a particular embodiment, the compound for use as defined above is of following formula (IVa):

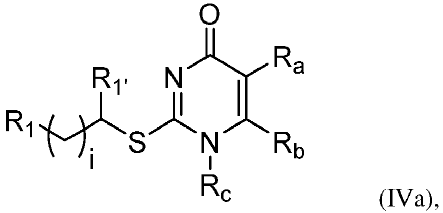

Wherein Ra, Rb, R_{c}, R₁, R_{1'} and i are as defined above, and in particular wherein:
Ra is hydrogen, aryl, in particular phenyl, or aralkyl, in particular benzyl, more particularly hydrogen or benzyl;
R_{b} is NR₄R₅, in particular NH2, or OH; or
Rₐ and R_{b} together with the main ring carbon atoms to which they are attached may form a fused benzo or heteroaryl ring optionally substituted by 1, 2, 3 or 4 substituents which may be the same or different selected from halogen, (C₁-C₄)-alkyl, CN, CF3, OCF3, COR2, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio,or mono to perfluoro-(C₁-C₄)-alkyl), in particular from (C₁-C₄)-alkyl and COOR2; or
Rₐ and R_{b} together with the main ring carbon atoms to which they are attached may form a fused heterocycle, in particular a fused azaheterocycle, optionally substituted by 1, 2, 3 or 4 substituents which may be the same or different selected from (C₁-C₄)-alkyl, oxo, aryl or heteroaryl ring which is further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy), (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy, more particularly from oxo, aryl and halogen;
said fused azaheterocycle optionally comprises at least one N-R_{a'} member;
R_{a'} is (C₁-C₆)-alkyl, aryl, aryl-(C₁-C₆)-alkyl, in particular an aryl-(C₁-C₂)-alkyl, said aryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
R₂ and R₃ are independently hydrogen or (C₁-C₂₀)-alkyl, for instance (C₁-C₄)-alkyl;
R_{2'} is R₂ or NR₄R₅;
R₄ is hydrogen, (C₁-C₁₂)-alkyl, preferably (C₁-C₆)-alkyl;
R₅ is hydrogen, (C₁-C₁₂)-alkyl, preferably (C₁-C₆)-alkyl, aryl, heteroaryl or said aryl or heteroaryl group being optionally fused with a cycloalkane, in particular a (C₅-C₈)-cycloalcane and being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl, more particularly from halogen, CN, COOR2; or
R₄ and R₅ together with the nitrogen to which they are attached form a 5- to 8 membered ring optionally containing one or more further heteroatoms selected from oxygen, nitrogen, notably as N-R_{a'} member, and sulphur, optionnaly fused with an aromatic ring, preferably a benzene ring, said rings being optionally substituted by one or two substituents selected from hydroxy, oxo, (C₁-C4)-alkyl, (C₁-C₄)-alkyl-CO, aryl, in particular phenyl, or aralkyl, in particular benzyl, for example morpholine or piperazine; or
R_{c} is (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, more particularly (C₁-C₆)-alkyl, aryl, or aryl-(C₁-C₆)-alkyl, said aryl or heteroaryl being further optionally substituted by 1, 2, 3 or 4 substituents which may be the same or different selected from (C₁-C₁₈)-alkyl, in particular (C₁-C6)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy, more particularly from halogen, (C₁-C₆)-alkoxy and CF3;
i is 0 or 1, in particular 0;
W is S, O, or CH2, in particular S;
R₁ is COOR2, COR_{2'}, OR_{3'}, an aryl or heteroaryl group, said group being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C4)-alkyl, mono to perfluoro-(C₁-C₄)-alkoxyaryl, and aryl-(C₁-C₄)-alkyl, more particularly halogen;
R_{1'} is H or (C₁-C₃)-alkyl, in particular H;
R_{3'} is an aryl or heteroaryl group, in particular an aryl, said group being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C4)-alkyl, mono to perfluoro-(C₁-C₄)-alkoxyaryl, and aryl-(C₁-C₄)-alkyl, more particularly being CF₃.

In a particular embodiment, the compound for use as defined above is of following formula (IVa): 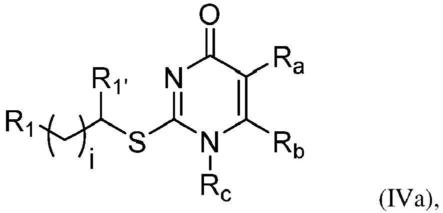 wherein:
Ra is hydrogen, aryl, in particular phenyl, or aralkyl, in particular benzyl or phenetyl, more particularly hydrogen or benzyl or phenetyl;
R_{b} is NR₄R₅, in particular NH2, or OH; or
Rₐ and R_{b} together with the main ring carbon atoms to which they are attached may form a fused benzo or heteroaryl ring, in particular a pyridine, optionally substituted by 1, 2, 3 or 4 substituents which may be the same or different selected from halogen, (C₁-C₄)-alkyl, CN, CF₃, OCF₃, COR₂, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio, or mono to perfluoro-(C₁-C₄)-alkyl), in particular from (C₁-C₄)-alkyl and COOR2; or
Rₐ and R_{b} together with the main ring carbon atoms to which they are attached may form a fused azaheterocycle, substituted by an oxo group, forming in particular a lactame with the azaheterocycle, Ra and R_{b} together with the main ring carbon atoms to which they are attached
forming in particular 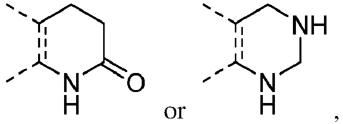 and optionnaly further by 1, 2, or 3 substituents which may be the same or different selected from (C₁-C₄)-alkyl, aryl or heteroaryl ring, in particular aryl, which is further optionally substituted by 1, 2, 3 or 4 substituents which may be the same or different selected from (C₁-C₁₈)-alkyl, in particular (C₁-C₆)-alkyl, (C₁-C₁₈₎-alkoxy, in particular (C₁-C₆)-alkoxy), (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy, more particularly from oxo, aryl and halogen;
said fused azaheterocycle optionally comprises at least one N-R_{a'} member;
Rₐ and R_{b} together with the main ring carbon atoms to which they are attached forming in particular 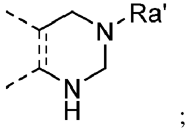
R_{a'} is (C₁-C₆)-alkyl, aryl, aryl-(C₁-C₆)-alkyl, in particular an aryl-(C₁-C₂)-alkyl, more particularly a benzyl or a phenethyl, said aryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
R₂ is hydrogen or (C₁-C₂₀)-alkyl, for instance (C₁-C₄)-alkyl;
R_{2'} is NR₄R₅;
R₄ is hydrogen, (C₁-C₁₂)-alkyl, preferably (C₁-C₆)-alkyl;
R₅ is (C₁-C₁₂)-alkyl, preferably (C₁-C₆)-alkyl, aryl, in particular phenyl, heteroaryl, or said aryl or heteroaryl group being optionally fused with a cycloalkane, in particular a (C₅-C₈)-cycloalcane, R₅ being in particular thiophene, 4,5,6,7-tetrahydrobenzo[*b*]thiophene, 4,5,6,7,8,9-hexahydrocycloocta[*b*]thiophene, thiazole or 4,5,6,7-tetrahydrobenzo[*d*]thiazole, and being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl, more particularly from halogen, CN, COOR2; or
R₄ and R₅ together with the nitrogen to which they are attached form a 5- to 8 membered ring optionally containing one or more further heteroatoms selected from oxygen, nitrogen, notably as N-R_{a'} member, and sulphur, optionnaly fused with an aromatic ring, preferably a benzene ring, R₄ and R₅ together with the nitrogen to which they are attached forming in particular a piperidine, a 1,2,3,4-tetrahydroquinoline ring or a piperazine, said rings being optionally substituted by one or two substituents selected from hydroxy, oxo, (C₁-C₄)-alkyl, (C₁-C₄)-alkyl-CO, aryl, in particular phenyl, or aralkyl, in particular benzyl, for example morpholine or piperazine; or
R_{c} is (C₁-C₆)-alkyl, aryl, in particular phenyl, aryl-(C₁-C₆)-alkyl, in particular an aryl-(C₁-C₂)-alkyl, more particularly benzyl or phenethyl, heteroaryl or heteroaryl-(C₁-C₆)-alkyl, in particular a heteroaryl-(C₁-C₂)-alkyl, more particularly (C₁-C₆)-alkyl, aryl, or aryl-(C₁-C₆)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy, more particularly from halogen, (C₁-C₆)-alkoxy and CF3;
i is 0 or 1, in particular 0;
W is S, O, or CH2, in particular S;
R₁ is COOR2, COR_{2'}, OR_{3'}, an aryl or heteroaryl group, in particular a phenyl or a 4*H*-pyrido[1,2-a]pyrimidin-4-one, said group being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl, more particularly halogen;
R_{1'} is H or (C₁-C₃)-alkyl, in particular H or Me, more particularly H;
R_{3'} is an aryl or heteroaryl group, in particular an aryl, more particularly a phenyl, said group being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl, more particularly being CF3.

In a particular embodiment, the compound for use as defined above is of following formula (IV):

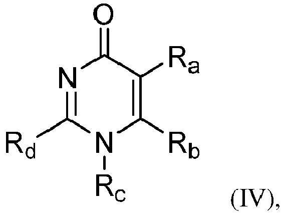

Wherein Ra and R_{b} are as defined above, Ra and R_{b} together bein in particular (CH₂)ₙ where n is 3 or 4, to form, with the main ring carbon atoms to which they are attached a fused 5-or 6-membered carbocyclic ring;
and wherein:
R_{c} is of following formula (A): 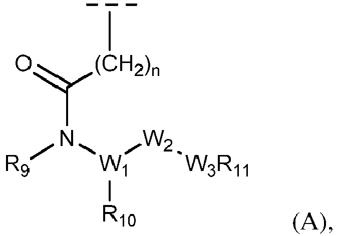
R₉ is hydrogen, (C₁-C₆)-alkyl which may be unsubstituted or substituted by 1, 2 or 3 substituents selected from hydroxy, halogen, OR2, COR2, carboxy, COOR2, CONR₄R₅, NR₄R₅, NR₄COR₅, mono-or di-(hydroxyl-(C₁-C₆)-alkyl)amino and N-hydroxy-(C₁-C₆)-alkyl-N-(C₁-C₆)-alkylamino, for example 1-piperidinoethyl; or
R₉ is Het-(C₀-C₄)-alkyl in which Het is a 5- to 7- membered heterocyclyl ring comprising N and optionally O or S, bonded through a carbon ring atom and in which N may be substituted by COR₂, COOR2, CONR₄R₅, or (C₁-C₆)-alkyl optionally substituted by 1, 2 or 3 substituents selected from hydroxy, halogen, OR2, COR2, carboxy, COOR2, CONR₄R₅, NR₄R₅, NR₄COR₅, for instance, piperidin-4-yl, pyrrolidin-3-yl;
R₁₀ is hydrogen or (C₁-C₃)-alkyl;
R₁₁ is an aryl or heteroaryl ring which is further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy), (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
W₁ is (CH₂)ₚ(O)_{q} in which p is 1, 2 or 3 and q is 0 or p is 2 or 3 and q is 1;
W2 is an arene diyl or heteroarene diyl group which is further optionally substituted by 1, 2, 3 or 4 substituents which may be the same or different selected from (C₁-C₁₈)-alkyl, in particular (C₁-C6)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl and mono to perfluoro-(C₁-C₄)-alkoxy;
W₃ is O or a bond;
Rd is of the following formula (B): 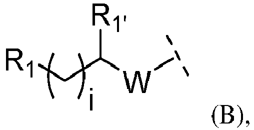
i is 0 or 1, in particular 0;
W is S, O, or CH2, in particular S;
R₁ is COOR2, COR_{2'}, OR_{3'}, an aryl or heteroaryl group, in particular aryl, more particularly phenyl, said group being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl;
R_{1'} is H or (C₁-C₃)-alkyl, in particular H.

In a particular embodiment, the compound for use as defined above is of following formula (V):

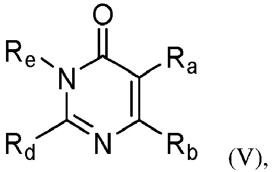

Wherein Ra, Rb, Rd, and Rₑ, are as defined above, and in particular wherein:
Rₐ and R_{b} together with the main ring carbon atoms to which they are attached form a fused benzo or heteroaryl ring optionally substituted by 1, 2, 3 or 4 substituents which may be the same or
different selected from halogen, (C₁-C₄)-alkyl, CN, CF3, OCF3, COR2, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio,or mono to perfluoro-(C₁-C₄)-alkyl);
R_{a'} is (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
R₂ and R₃ are independently hydrogen or (C₁-C₂₀)-alkyl, for instance (C₁-C₄)-alkyl;
R_{2'} is R₂ or NR₄R₅;
R₄ is hydrogen, (C₁-C₁₂)-alkyl, preferably (C₁-C₆)-alkyl, (C₁-C₁₂)-alkyl-R_{4'}, preferably (C₁-C₆)-alkyl-R_{4'};
R4 being in particular (C₁-C₆)-alkyl-R_{4'};
R_{4'} is NH2, mono- or di-(C₁-C₃)-alkylamino, in particular di-(C₁-C₃)-alkylamino;
R₅ is hydrogen, (C₁-C₁₂)-alkyl, preferably (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl group being optionally fused with a cycloalkane, in particular a (C₅-C₈)-cycloalcane or a 5- to 8- membered heterocycle comprising N and optionally O or S, and being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl, more particularly halogen; or
R₅ being in particular arly or substituted aryl; or
R₄ and R₅ together with the nitrogen to which they are attached form a 5- to 8 membered ring optionally containing one or more further heteroatoms selected from oxygen, nitrogen, notably as N-R_{a'} member, and sulphur, optionnaly fused with an aromatic ring, preferably a benzene ring, said rings being optionally substituted by one or two substituents selected from hydroxy, oxo, (C₁-C4)-alkyl, (C₁-C₄)-alkyl-CO, aryl, in particular phenyl, or aralkyl, in particular benzyl, for example morpholine or piperazine;
Rd is H or (C₁-C₆)-alkyl, in particular H;
Rₑ is (C₁-C₆)-alkyl-R_{e'};
R_{e'} is COR2, carboxy, COOR2, CONR₄R₅, in particular CONR₄R₅.

In a particular embodiment, the compound for use as defined above is of following formula (V): 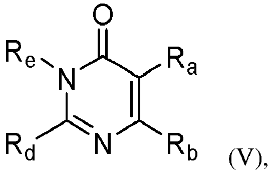 wherein:
Rₐ and R_{b} together with the main ring carbon atoms to which they are attached form a fused benzo or heteroaryl ring, in particular a benzo ring, optionally substituted by 1, 2, 3 or 4 substituents which may be the same or different selected from halogen, (C₁-C₄)-alkyl, CN, CF₃, OCF₃, COR₂, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio, or mono to perfluoro-(C₁-C₄)-alkyl);
R₄ is hydrogen, (C₁-C₁₂)-alkyl, preferably (C₁-C₆)-alkyl, (C₁-C₁₂)-alkyl-R_{4'}, preferably (C₁-C₆)-alkyl-R_{4'};
R4 being in particular (C₁-C₆)-alkyl-R_{4'};
R_{4'} is NH2, mono- or di-(C₁-C₃)-alkylamino, in particular di-(C₁-C₃)-alkylamino;
R₅ is aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, R₅ being more particularly phenyl, said aryl or heteroaryl group being optionally fused with a cycloalkane, in particular a (C₅-C₈)-cycloalcane or a 5- to 8- membered heterocycle comprising N and optionally O or S, and being optionally substituted by 1, 2, 3 or 4 groups independently selected from (C₁-C₁₈)-alkyl, in particular (C₁-C6)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl, more particularly halogen; or
R₅ being in particular arly or substituted aryl;
Rd is H or (C₁-C₆)-alkyl, in particular H;
Rₑ is (C₁-C₆)-alkyl-R_{e'}, in particular -CH₂-R_{e'};
R_{e'} is CONR₄R₅.

In a particular embodiment, the compound for use as defined above is of following formula (Va):

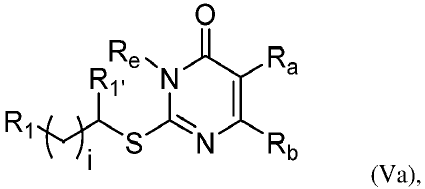

Wherein Rₐ, Rb, Rₑ, R₁, R₁, and i are as defined above, and in particular wherein:
Rₐ and R_{b} together with the main ring carbon atoms to which they are attached may form a fused benzo or heteroaryl ring optionally substituted by 1, 2, 3 or 4 substituents which may be the same or different selected from halogen, (C₁-C₄)-alkyl, CN, CF3, OCF3, COR2, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio, or mono to perfluoro-(C₁-C₄)-alkyl);
R₂ and R₃ are independently hydrogen or (C₁-C₂₀)-alkyl, for instance (C₁-C₄)-alkyl;
R_{2'} is R₂ or NR₄R₅;
R₄ is hydrogen;
R₅ is aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl group being optionally fused with a cycloalkane, in particular a (C₅-C₈)-cycloalcane or a 5- to 8- membered heterocycle comprising N and optionally O or S, and being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl, more particularly from (C₁-C₁₈)-alkyl; or
i is 0 or 1, in particular 0;
W is S, O, or CH2, in particular S;
R₁ is an aryl or heteroaryl group, in particular an aryl, said group being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl, more particularly from halogen;
R_{1'} is H or (C₁-C₃)-alkyl, in particular H;
Rₑ is (C₁-C₆)-alkyl-R_{e'};
R_{e'} is COR2, carboxy, COOR2, CONR₄R₅, in particular CONR₄R₅.

In a particular embodiment, the compound for use as defined above is of following formula (Va): 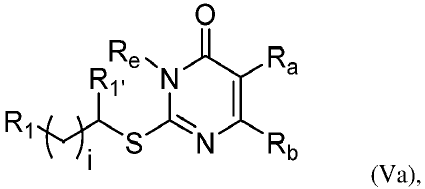 wherein:
Rₐ and R_{b} together with the main ring carbon atoms to which they are attached may form a fused benzo or heteroaryl ring, in particular a benzo ring, optionally substituted by 1, 2, 3 or 4 substituents which may be the same or different selected from halogen, (C₁-C₄)-alkyl, CN, CF3, OCF3, COR2, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio, or mono to perfluoro-(C₁-C₄)-alkyl);
R₄ is hydrogen;
R₅ is aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, R₅ being more particularly benzyl or phenethyl, said aryl or heteroaryl group being optionally fused with a cycloalkane, in particular a (C₅-C₈)-cycloalcane or a 5- to 8- membered heterocycle comprising N and optionally O or S, and being optionally substituted by 1, 2, 3 or 4 groups independently selected from (C₁-C₁₈)-alkyl, in particular (C₁-C6)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl, more particularly from (C₁-C₁₈)-alkyl; or
i is 0 or 1, in particular 0;
W is S, O, or CH2, in particular S;
R₁ is an aryl or heteroaryl group, in particular an aryl, more particularly a phenyl, said group being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl, more particularly from halogen;
Rₑ is (C₁-C₆)-alkyl-R_{e'};
R_{e'} is CONR₄R₅.

In a particular embodiment, the compound for use as defined above is of following formula (VI): 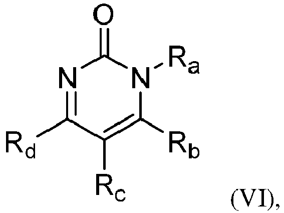 wherein Rₐ, R_{b}, R_{c}, and R_{d} are as defined above.

In a particular embodiment, the compound for use as defined above is of following formula (VIa):

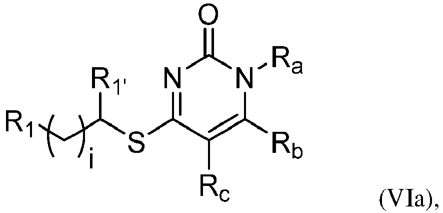

Wherein Rₐ, Rb, R_{c}, R₁, R_{1'} and i are as defined above, and in particular wherein:
Rₐ is NR₄R₅, (C₁-C₄)-alkoxy-(C₁-C₆)-alkyl, aryl-(C₁-C₆)-alkyl, in particular an aryl-(C₁-C₂)-alkyl, heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
R_{a'} is (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy, R_{a'} being more particularly (C₁-C₆)-alkyl;
R₂ and R₃ are independently hydrogen or (C₁-C₂₀)-alkyl, for instance (C₁-C₄)-alkyl;
R_{2'} is R₂ or NR₄R₅;
R₄ is hydrogen, (C₁-C₁₂)-alkyl, preferably (C₁-C₆)-alkyl, (C₁-C₁₂)-alkyl-R_{4'}, preferably (C₁-C₆)-alkyl-R_{4'};
R_{4'} is NH₂, mono- or di-(C₁-C₃)-alkylamino;
R₅ is hydrogen, (C₁-C₁₂)-alkyl, preferably (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl group being optionally fused with a cycloalkane, in particular a (C₅-C₈)-cycloalcane or a 5- to 8- membered heterocycle comprising N and optionally O or S, and being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl; or
R₄ and R₅, preferably, together with the nitrogen to which they are attached form a 5- to 8 membered ring optionally containing one or more further heteroatoms selected from oxygen, nitrogen, notably as N-R_{a'} member, and sulphur, optionnaly fused with an aromatic ring, preferably a benzene ring, said rings being optionally substituted by one or two substituents selected from hydroxy, oxo, (C₁-C₄)-alkyl, (C₁-C₄)-alkyl-CO, aryl, in particular phenyl, or aralkyl, in particular benzyl, for example morpholine or piperazine; or
R_{c} and R_{b} together may be (CH2)ₙ where n is 3 or 4, to form, with the main ring carbon atoms to which they are attached a fused 5-or 6-membered carbocyclic ring;
i is 0 or 1, in particular 0;
W is S, O, or CH2, in particular S;
R₁ is an aryl or heteroaryl group, in particular aryl, said group being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl, more particularly from halogen and CF₃;
R_{1'} is H or (C₁-C₃)-alkyl, in particular H.

In a particular embodiment, the compound for use as defined above is of following formula (VIa): 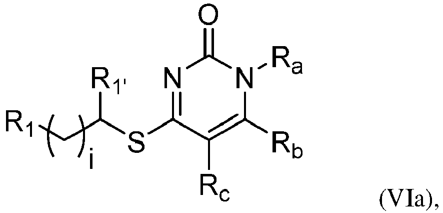 wherein:
Rₐ is NR₄R₅, (C₁-C₄)-alkoxy-(C₁-C₆)-alkyl, aryl-(C₁-C₆)-alkyl, in particular an aryl-(C₁-C₂)-alkyl, more particularly benzyl or phenethyl, heteroaryl-(C₁-C₆)-alkyl, in particular a heteroaryl-(C₁-C₂)-alkyl, said heteroaryl being more particularly pyridyl or furanyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
R_{a'} is (C₁-C₆)-alkyl;
R₄ and R₅ together with the nitrogen to which they are attached form a 5- to 8 membered ring optionally containing one or more further heteroatoms selected from oxygen, nitrogen, notably as N-R_{a'} member, and sulphur, optionnaly fused with an aromatic ring, preferably a benzene ring, said rings being optionally substituted by one or two substituents selected from hydroxy, oxo, (C₁-C4)-alkyl, (C₁-C₄)-alkyl-CO, aryl, in particular phenyl, or aralkyl, in particular benzyl, for example morpholine or piperazine, R₄ and R₅ together with the nitrogen to which they are attached preferably form a piperazine or a R_{a'} substituted piperazine; or
R_{c} and R_{b} together may be (CH₂)ₙ where n is 3 or 4, to form, with the main ring carbon atoms to which they are attached a fused 5-or 6-membered carbocyclic ring;
i is 0 or 1, in particular 0;
W is S, O, or CH2, in particular S;
R₁ is an aryl or heteroaryl group, in particular aryl, more particularly a phenyl, said group being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl, more particularly from halogen and CF3.

In a particular embodiment, the compound for use as defined above is of following formula (VII):

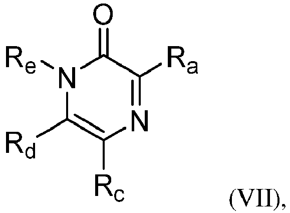

Wherein Rₐ, R_{c}, Rd, and Rₑ are as defined above, and in particular wherein:
Rₐ is hydrogen or (C₁-C₃)-alkyl, in particular hydrogen;
R_{a'} is (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
R₂ and R₃ are independently hydrogen or (C₁-C₂₀)-alkyl, for instance (C₁-C₄)-alkyl;
R_{2'} is R₂ or NR₄R₅;
R₄ is hydrogen, (C₁-C₁₂)-alkyl, preferably (C₁-C₆)-alkyl, (C₁-C₁₂)-alkyl-R_{4'}, preferably (C₁-C₆)-alkyl-R_{4'};
R_{4'} is NH₂, mono- or di-(C₁-C₃)-alkylamino;
R₅ is hydrogen, (C₁-C₁₂)-alkyl, preferably (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl group being optionally fused with a cycloalkane, in particular a (C₅-C₈)-cycloalcane or a 5- to 8- membered heterocycle comprising N and optionally O or S, and being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl; or
preferably, R₄ and R₅ together with the nitrogen to which they are attached form a 5- to 8 membered ring optionally containing one or more further heteroatoms selected from oxygen, nitrogen, notably as N-R_{a'} member, and sulphur, optionnaly fused with an aromatic ring, preferably a benzene ring, said rings being optionally substituted by one or two substituents selected from hydroxy, oxo, (C₁-C₄)-alkyl, (C₁-C₄)-alkyl-CO, aryl, in particular phenyl, or aralkyl, in particular benzyl, for example morpholine or piperazine;
R_{c} and R_{d} may together with the main ring carbon atoms to which they are attached form a fused benzo or heteroaryl ring, in particular benzo, optionally substituted by 1, 2, 3 or 4 substituents which may be the same or different selected from halogen, (C₁-C₄)-alkyl, CN, CF₃, OCF₃, COR₂, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio, or mono to perfluoro-(C₁-C₄)-alkyl);
Rₑ is (C₁-C₆)-alkyl-R_{e'};
R_{e'} is COR2, carboxy, COOR2, CONR₄R₅, in particular CONR₄R₅.

In a particular embodiment, the compound for use as defined above is of following formula (VII): 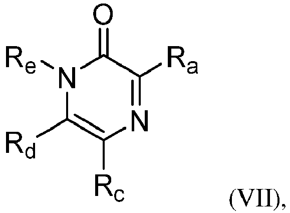 wherein:
Rₐ is hydrogen or (C₁-C₃)-alkyl, in particular hydrogen;
R_{a'} is (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, more particularly benzyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
R₂ and R₃ are independently hydrogen or (C₁-C₂₀)-alkyl, for instance (C₁-C₄)-alkyl;
R_{2'} is R₂ or NR₄R₅;
R₄ and R₅ together with the nitrogen to which they are attached form a 5- to 8 membered ring optionally containing one or more further heteroatoms selected from oxygen, nitrogen, notably as N-R_{a'} member, and sulphur, optionnaly fused with an aromatic ring, preferably a benzene ring, said rings being optionally substituted by one or two substituents selected from hydroxy, oxo, (C₁-C4)-alkyl, (C₁-C₄)-alkyl-CO, aryl, in particular phenyl, or aralkyl, in particular benzyl, for example morpholine or piperazine, R₄ and R₅ together with the nitrogen to which they are attached forming in particular a piperazine or a *N*-R_{a'} substituted piperazine, optionally further substituted by an aryl, for example a phenyl;
R_{c} and R_{d} together with the main ring carbon atoms to which they are attached form a fused benzo or heteroaryl ring, in particular a benzo ring, optionally substituted by 1, 2, 3 or 4 substituents which may be the same or different selected from halogen, (C₁-C₄)-alkyl, CN, CF₃, OCF₃, COR₂, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio, or mono to perfluoro-(C₁-C₄)-alkyl);
Rₑ is (C₁-C₆)-alkyl-R_{e'}, in particular -CH₂-R_{e'};
R_{e'} is CONR₄R₅.

In a particular embodiment, the compound for use as defined above is of following formula (VIII):

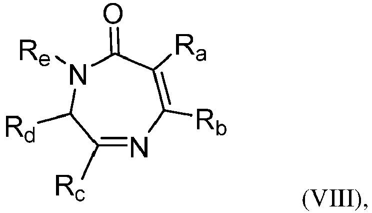

Wherein Rₐ, R_{b}, R_{c}, R_{d}, and Rₑ are as defined above, and in particular wherein:
Rₐ and R_{b} together with the main ring carbon atoms to which they are attached may form a fused benzo or heteroaryl ring, in particular benzo, optionally substituted by 1, 2, 3 or 4 substituents which may be the same or different selected from halogen, (C₁-C₄)-alkyl, CN, CF₃, OCF₃, COR₂, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio, or mono to perfluoro-(C₁-C₄)-alkyl);
said fused azaheterocycle optionally comprises at least one N-R_{a'} member;
R_{a'} is (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
R₂ and R₃ are independently hydrogen or (C₁-C₂₀)-alkyl, for instance (C₁-C₄)-alkyl;
R_{2'} is R₂ or NR₄R₅;
R₄ is hydrogen, (C₁-C₁₂)-alkyl, preferably (C₁-C₆)-alkyl, (C₁-C₁₂)-alkyl-R_{4'}, preferably (C₁-C₆)-alkyl-R_{4'};
R_{4'} is NH₂, mono- or di-(C₁-C₃)-alkylamino;
R₅ is hydrogen, (C₁-C₁₂)-alkyl, preferably (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl group being optionally fused with a cycloalkane, in particular a (C₅-C₈)-cycloalcane or a 5- to 8- membered heterocycle comprising N and optionally O or S, and being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl; or
preferably, R₄ and R₅ together with the nitrogen to which they are attached form a 5- to 8 membered ring optionally containing one or more further heteroatoms selected from oxygen, nitrogen, notably as N-R_{a'} member, and sulphur, optionnaly fused with an aromatic ring, preferably a benzene ring, said rings being optionally substituted by one or two substituents selected from hydroxy, oxo, (C₁-C₄)-alkyl, (C₁-C₄)-alkyl-CO, aryl, in particular phenyl, or aralkyl, in particular benzyl, for example morpholine or piperazine;
R_{c} is OH or (C₁-C₁₈)-alkoxy, in particular (C₁-C₆)-alkoxy, R_{c} being notably OH;
Rd is of the following formula (B): 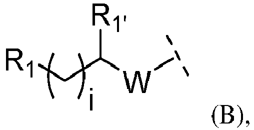
i is 0 or 1, in particular 0;
W is S, O, or CH2, in particular CH2;
R₁ is COOR₂ or COR_{2'},
R_{1'} is H or (C₁-C₃)-alkyl;
Rₑ is H or (C₁-C₆)-alkyl, in particular H.

In a particular embodiment, the compound for use as defined above is of following formula (Villa): 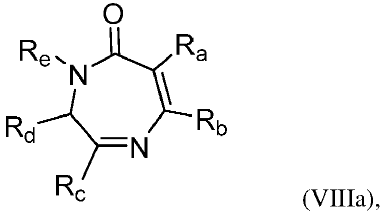
Rₐ and R_{b} together with the main ring carbon atoms to which they are attached form a fused benzo ring, optionally substituted by 1, 2, 3 or 4 substituents which may be the same or different selected from halogen, (C₁-C₄)-alkyl, CN, CF₃, OCF₃, said benzo ring being preferably unsubstituted;
R_{c} is OH or (C₁-C₁₈)-alkoxy, in particular (C₁-C₆)-alkoxy, R_{c} being notably OH;
Rd is of the following formula (B): 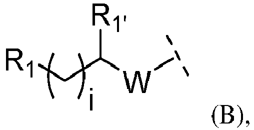
i is 0 or 1, in particular 0;
W is S, O, or CH2, in particular CH2;
R₁ is COR_{2'};
R_{1'} is H or (C₁-C₃)-alkyl, in particular H;
R_{2'} is NR₄R₅;
R₄ is hydrogen, (C₁-C₁₂)-alkyl, preferably (C₁-C₆)-alkyl, (C₁-C₁₂)-alkyl-R_{4'}, preferably (C₁-C₆)-alkyl-R_{4'};
R_{4'} is NH₂, mono- or di-(C₁-C₃)-alkylamino;
Rs is hydrogen, (C₁-C₁₂)-alkyl, preferably (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl group being optionally fused with a cycloalkane, in particular a (C₅-C₈)-cycloalcane or a 5- to 8- membered heterocycle comprising N and optionally O or S, and being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl; or
preferably, R₄ and R₅ together with the nitrogen to which they are attached form a 5- to 8 membered ring optionally containing one or more further heteroatoms selected from oxygen, nitrogen, notably as N-R_{a'} member, and sulphur, optionnaly fused with an aromatic ring, preferably a benzene ring, said rings being optionally substituted by one or two substituents selected from hydroxy, oxo, (C₁-C₄)-alkyl, (C₁-C₄)-alkyl-CO, aryl, in particular phenyl, or aralkyl, in particular benzyl, for example morpholine or piperazine;
R_{a'} is (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
Rₑ is H or (C₁-C₆)-alkyl, in particular H.

In a particular embodiment, the compound for use as defined above is of following formula (Villa): 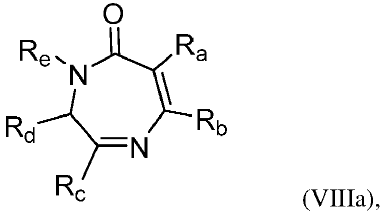
Rₐ and R_{b} together with the main ring carbon atoms to which they are attached form a fused benzo ring, optionally substituted by 1, 2, 3 or 4 substituents which may be the same or different selected from halogen, (C₁-C₄)-alkyl, CN, CF₃, OCF₃, said benzo ring being preferably unsubstituted;
R_{c} is OH or (C₁-C₁₈)-alkoxy, in particular (C₁-C₆)-alkoxy, R_{c} being notably OH;
Rd is of the following formula (B): 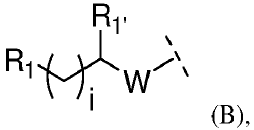
i is 0 or 1, in particular 0;
W is S, O, or CH2, in particular CH2;
R₁ is COR_{2'};
R_{1'} is H or (C₁-C₃)-alkyl, in particular H;
R_{2'} is NR₄R₅;
R₄ and R₅ together with the nitrogen to which they are attached form a 5- to 8 membered ring optionally containing one further nitrogen heteroatom as N-R_{a'} member;
R_{a'} is aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
Rₑ is H or (C₁-C₆)-alkyl, in particular H.

In a particular embodiment, the compound for use as defined above is of one of the following formulae:

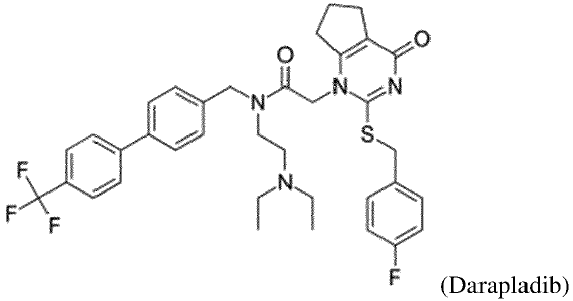

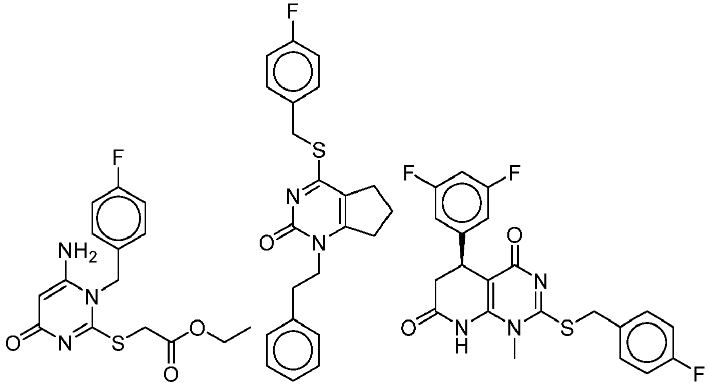

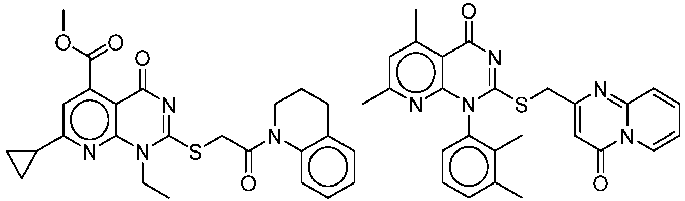

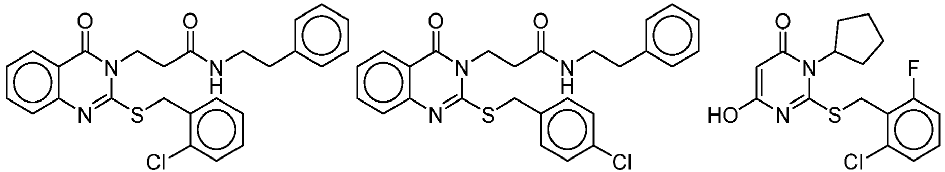

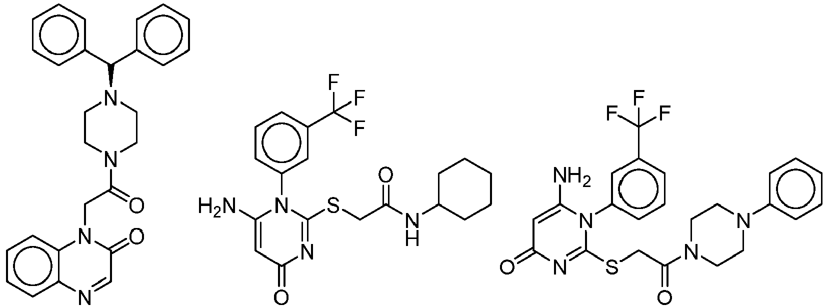

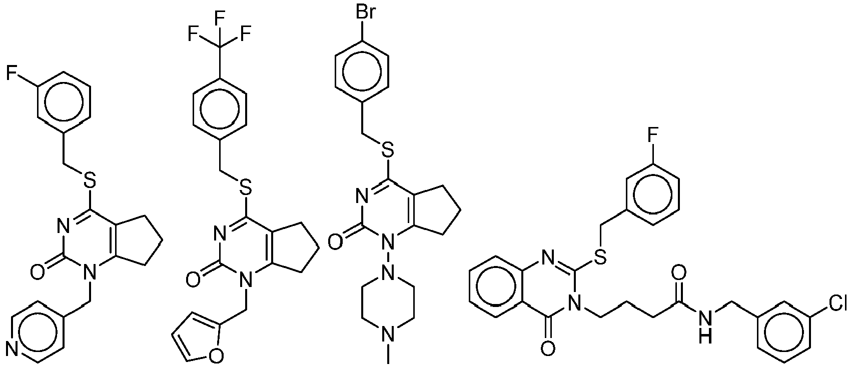

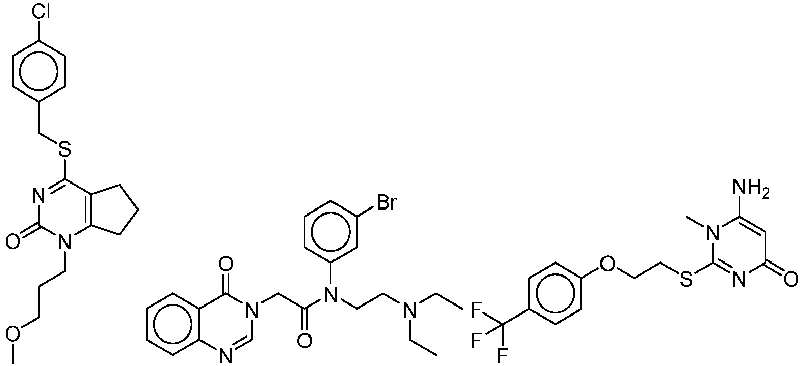

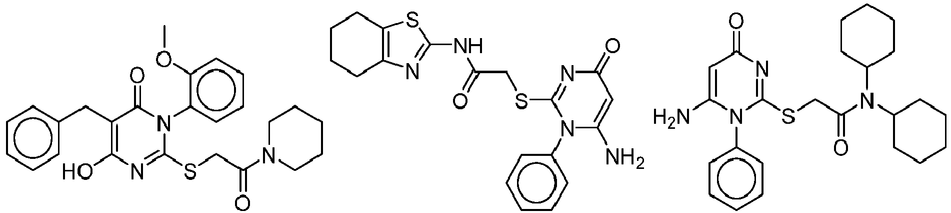

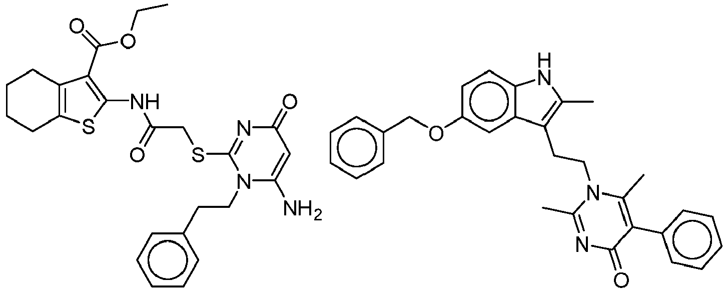

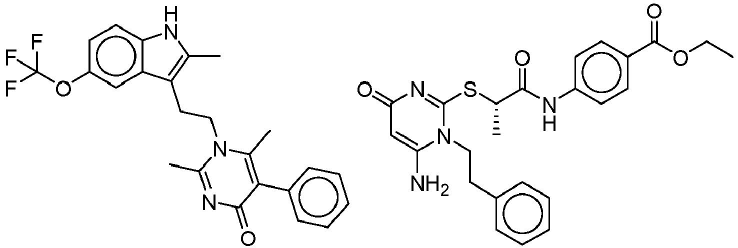

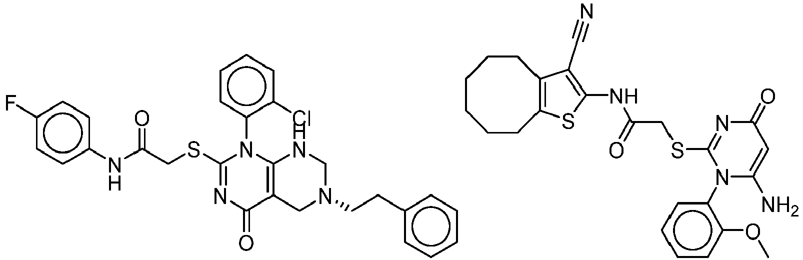

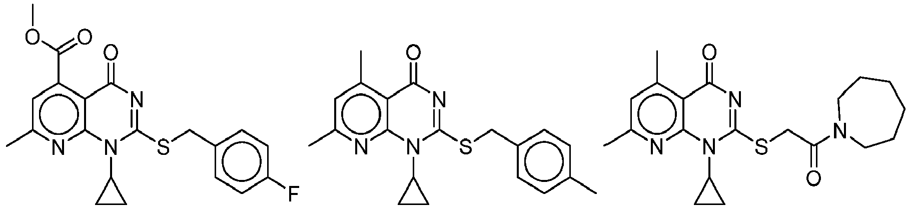

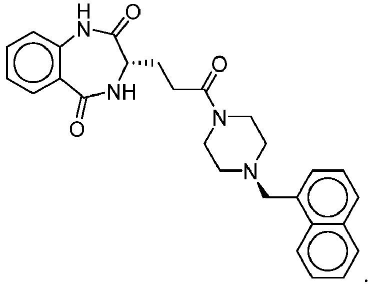

In a particular embodiment, malaria is caused by a drug sensitive or drug resistant malaria parasite, in particular a drug resistant malaria parasite, more particularly an artemisinin-resistant malaria parasite.

In a particular embodiment, the drug-sensitive or drug-resistant malaria parasite is an artemisin-sensitive, an artemisinin-resistant; a chloroquine-sensitive; a chloroquine-resistant; a chloroquine and pyrimethamine-sensitive; a chloroquine and pyrimethamine-resistant; a chloroquine, pyrimethamine and mefloquine-sensitive; a chloroquine, pyrimethamine and mefloquine-resistant; a chloroquine, pyrimethamine, mefloquine and artemisinin-sensitive; a chloroquine, pyrimethamine, mefloquine and artemisinin-resistant; a chloroquine, pyrimethamine, piperaquine and artemisinin-sensitive; a chloroquine, pyrimethamine, piperaquine and artemisinin-resistant; or a chloroquine, pyrimethamine, piperaquine, amodiaquine and artemisinin-resistant malaria parasite.

In a more particular embodiment, the drug-sensitive or drug-resistant malaria parasite is an artemisinin-resistant; a chloroquine, pyrimethamine, mefloquine and artemisinin-resistant; a chloroquine, pyrimethamine, piperaquine and artemisinin-resistant; or a chloroquine, pyrimethamine, piperaquine, amodiaquine and artemisinin-resistant malaria parasite.

In a particular embodiment, malaria is caused by infection with Plasmodium, in particular by *Plasmodium falciparum, P. vivax, P. ovale,* or *P. malariae, P. knowlesi* more particularly by *Plasmodium falciparum.*

In another aspect, the invention concerns a combination of a compound as defined above, with at least one other antimalarial agent for use in the treatment of malaria with simultaneous administration, separate or spread out over time.

In a particular embodiment, the other antimalarial agent is selected from chloroquine, artemesin, qinghaosu, 8-aminoquinoline, amodiaquine, arteether, artemether, artemisinin, artesunate, artesunic acid, artelinic acid, atovaquone, azithromycine, biguanide, chloroquine phosphate, chlorproguanil, cycloguanil, dapsone, desbutyl halofantrine, desipramine, doxycycline, dihydrofolate reductase inhibitors, dipyridamole, halofantrine, haloperidol, hydroxychloroquine sulfate, imipramine, mefloquine, penfluridol, phospholipid inhibitors, primaquine, proguanil, pyrimethamine, pyronaridine, quinine, quinidine, quinacrineartemisinin, sulfonamides, sulfones, sulfadoxine, sulfalene, tafenoquine, tetracycline, tetrandine, triazine, salts and mixture thereof, in particular artemisinin.

In another aspect, the present invention concerns a method for the treatment of malaria comprising the administration to a person in need thereof of an effective dose of a compound as defined above.

### Definitions

The following terms and expressions contained herein are defined as follows:
As used herein, a range of values in the form "x-y" or "x to y", or "x through y", include integers x, y, and the integers there between. For example, the phrases "1-6", or "1 to 6" or "1 through 6" are intended to include the integers 1, 2, 3, 4, 5, and 6. Preferred embodiments include each individual integer in the range, as well as any subcombination of integers. For example, preferred integers for "1-6" can include 1, 2, 3, 4, 5, 6, 1-2, 1-3, 1-4, 1-5, 2-3, 2-4, 2-5, 2-6, etc.

As used herein, the term "patient" or "subject" refers to a warm blooded animal such as a mammal, preferably a human, or a human child, which is afflicted with, or has the potential to be afflicted with one or more diseases and conditions described herein.

By drug-susceptible malaria is meant a case wherein malaria is treated or prevented when the recommended drug dosage is used.

By drug-resistant malaria is meant a case wherein malaria is not treated or prevented when the recommended drug dosage is used.

For the purpose of the invention, the term "pharmaceutically acceptable" is intended to mean what is useful to the preparation of a pharmaceutical composition, and what is generally safe and non-toxic, for a pharmaceutical use.

The term "pharmaceutically acceptable salt or solvate" is intended to mean, in the framework of the present invention, a salt or solvate of a compound which is pharmaceutically acceptable, as defined above, and which possesses the pharmacological activity of the corresponding compound.

The pharmaceutically acceptable salts comprise:
(1) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, and
(2) base addition salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

Acceptable solvates for the therapeutic use of the compounds of the present invention include conventional solvates such as those formed during the last step of the preparation of the compounds of the invention due to the presence of solvents. As an example, mention may be made of solvates due to the presence of water (these solvates are also called hydrates) or ethanol.

It is recognized that compounds of the present invention may exist in various stereoisomeric forms. As such, the compounds of the present invention include both diastereomers and enantiomers. The compounds are normally prepared as racemates and can conveniently be used as such, but individual enantiomers can be isolated or synthesized by conventional techniques if so desired. Such racemates and individual enantiomers and mixtures thereof form part of the present invention.

It is well known in the art how to prepare and isolate such optically active forms. Specific stereoisomers can be prepared by stereospecific synthesis using enantiomerically pure or enantiomerically enriched starting materials. The specific stereoisomers of either starting materials or products can be resolved and recovered by techniques known in the art, such as resolution of racemic forms, normal, reverse-phase, and chiral chromatography, recrystallization, enzymatic resolution, or fractional recrystallization of addition salts formed by reagents used for that purpose. Useful methods of resolving and recovering specific stereoisomers described in Eliel, E. L.; Wilen, S.H. Stereochemistry of Organic Compounds; Wiley: New York, 1994, and Jacques, J, et al. Enantiomers, Racemates, and Resolutions; Wiley: New York, 1981, each incorporated by reference herein in their entireties.

It is also specified that all the formulae defined in the present specification include all the possible resonance structures, being in particular noted that the structures represented herein may not be the most stable resonance structures.

The term "alkyl", as used in the present invention, refers in particular to a straight or branched saturated hydrocarbon chain, or a saturated or partially saturated mono- or bicyclic alkyl ring system.

The term "alkyl" thus includes the term "cycloalkyl", which refers in particular to a saturated or partially saturated mono- or bicyclic alkyl ring system.

The term "(C₁-C_{X})alkyl", as used in the present invention, refers in particular to a straight or branched saturated hydrocarbon chain containing from 1 to X carbon atoms.

The term "(C₁-C₇)alkyl", includs, but is not limited to, methyl, ethyl, n-propyl, iso-propyl, n-butyl, iso-butyl, sec-butyl, t-butyl, n-pentyl, n-hexyl, n-heptly and the like, or a (C₃-C₇)cycloalkyl, such as cyclopropyl, cyclobutyl, cyclopentyl, cyclohexyl, cycloheptyl, cyclooctyl, pinenyl, adamantanyl and the like.

The term "alcoxy" as used in the present invention, refers in particular to alkyl-O- group, wherein the term "alkyl" is as defined above.

The term "alkylthio" as used in the present invention, refers in particular to alkyl-O- group, wherein the term "alkyl" is as defined above.

As used herein, the term "aryl" refers to a substituted or unsubstituted, mono- or bicyclic hydrocarbon aromatic ring system having 6 to 10 ring carbon atoms. Examples include phenyl and naphthyl. Preferred aryl groups include unsubstituted or substituted phenyl and naphthyl groups. Included within the definition of "aryl" are fused ring systems, including, for example, ring systems in which an aromatic ring is fused to a cycloalkyl ring. Examples of such fused ring systems include, for example, indane, indene, and tetrahydronaphthalene.

As used herein, the term "aralkyl" refers to a aryl alkyl group of formula Ar-alkyl-, wherein "aryl" and "alkyl" are as defined above, Ar being in particular Ph, alkyl being in particular Me (-CH₂-).

As used herein, the term "heteroaryl" refers to an aromatic group containing 5 to 10 ring carbon atoms in which one or more ring carbon atoms are replaced by at least one hetero atom such as -O-, -N-, or -S-. Examples of heteroaryl groups include pyrrolyl, furanyl, thienyl, pirazolyl, imidazolyl, thiazolyl, isothiazolyl, isoxazolyl, oxazolyl, oxathiolyl, oxadiazolyl, triazolyl, oxatriazolyl, furazanyl, tetrazolyl, pyridyl, pyrazinyl, pyrimidinyl, pyridazinyl, triazinyl, indolyl, isoindolyl, indazolyl, benzofuranyl, isobenzofuranyl, purinyl, quinazolinyl, quinolyl, isoquinolyl, benzoimidazolyl, benzothiazolyl, benzothiophenyl, thianaphthenyl, benzoxazolyl, benzisoxazolyl, cinnolinyl, phthalazinyl, naphthyridinyl, and quinoxalinyl. Included within the definition of "heteroaryl" are fused ring systems, including, for example, ring systems in which an aromatic ring is fused to a heterocycloalkyl ring. Examples of such fused ring systems include, for example, phthalamide, phthalic anhydride, indoline, isoindoline, tetrahydroisoquinoline, chroman, isochroman, chromene, and isochromene. Also included within the definition of "heteroaryl" are aromatic oxo-(5 to 10)-ring carbon groups in which one or more ring carbon atoms are replaced by at least one hetero atom such as -O-, -N-, or -S-, being for example 4H-pyrido[1,2-a]pyrimidin-4-one.

The term "aryl-alkyl", as used in the present invention, refers to an aryl group as defined above bound to the molecule via an alkyl group as defined above. In particular, the aryl-alkyl group is a benzyl or phenetyl group.

The term "heteroaryl-alkyl", as used in the present invention, refers to a heteroaryl group as defined above bound to the molecule via an alkyl group as defined above.

The term "halogen", as used in the present invention, refers in particular to a fluorine, bromine, chlorine or iodine atom.

The term " alkylcarbonyl", as used in the present invention, refers in particular to alky-C(=O)- group, wherein " "alkyl" is as defined above.

The term " alkylcarbonylamino-alkyl ", as used in the present invention, refers in particular to an alkyl-C(=O)-NH-alkyl- group, wherein " "alkyl" is as defined above.

The term " alkylsulphinyl", as used in the present invention, refers in particular to alky-S(=O)- group, wherein " "alkyl" is as defined above.

The term "alkylsulphonylamino-alkyl", as used in the present invention, refers in particular to an alkyl-S(=O)₂-NH-alkyl- group, wherein " "alkyl" is as defined above.

### Synthesis

The compounds of the present invention may be prepared in a number of methods well known to those skilled in the art, including, but not limited to those described below, or through modifications of these methods by applying standard techniques known to those skilled in the art of organic synthesis. The appropriate modifications and substitutions will be readily apparent and well known or readily obtainable from the scientific literature to those skilled in the art. In particular, such methods can be found in R.C. Larock, Comprehensive Organic Transformations, Wiley-VCH Publishers, 1999.

All processes disclosed in association with the present invention are contemplated to be practiced on any scale, including milligram, gram, multigram, kilogram, multikilogram or commercial industrial scale.

It will be appreciated that the compounds of the present invention may contain one or more asymmetrically substituted carbon atoms, and may be isolated in optically active or racemic forms. Thus, all chiral, diastereomeric, racemic forms, isomeric forms of a structure are intended, unless the specific stereochemistry or isomeric form is specifically indicated. It is well-known in the art how to prepare and isolate such optically active forms. For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from chiral starting materials or by deliberate synthesis of target chiral centers.

Compounds of the present invention are commercially available or, if not, may be prepared by a variety of synthetic routes, for example by synthetic routes that are analogous to the synthetic routes to said commercially available compounds. The reagents and starting materials are commercially available, or readily synthesized by well-known techniques by one of ordinary skill in the arts. All substituents, unless otherwise indicated, are as previously defined.

In the reactions described hereinafter, it may be necessary to protect reactive functional groups, for example hydroxy, amino, imino, thio or carboxy groups, where these are desired in the final product, to avoid their unwanted participation in the reactions. Conventional protecting groups may be used in accordance with standard practice, for examples see T.W. Greene and P. G. M. Wuts in Protective Groups in Organic Chemistry, 3rd ed., John Wiley and Sons, 1999; J. F. W. McOmie in Protective Groups in Organic Chemistry, Plenum Press, 1973.

The reagents and starting materials are commercially available, or readily synthesized by well-known techniques by one of ordinary skill in the arts.

In particular, the compounds defined above are obtained according to the procedures described in patent applications EP 1 686 119 and EP 1 263 740, or analogously to these procedures.

### FIGURES

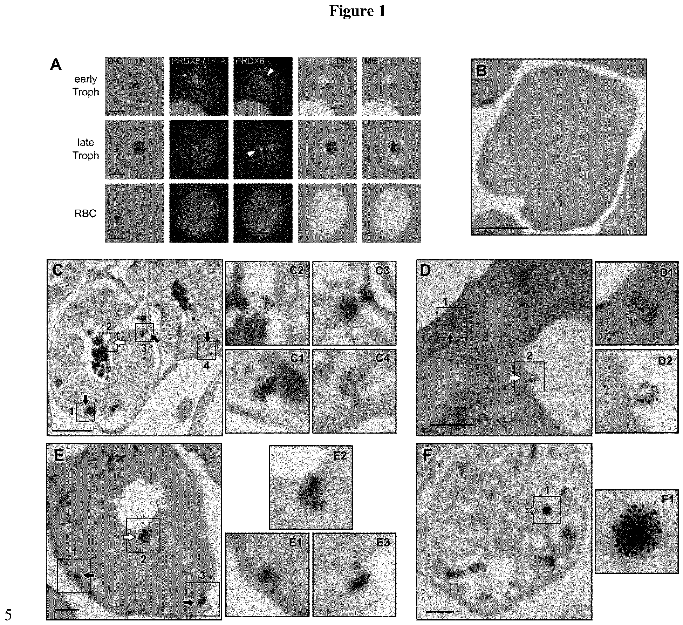

**Figure 1** shows that human PRDX6 is internalized by *P. falciparum* along with hemoglobin during HCCU (host cell cytosol uptake). (A) Immuno-fluorescence microscopy shows even cytosolic staining of PRDX6 in uninfected RBCs. In early and late trophozoites, multiple PRDX6 foci (white arrow heads) were observed adjacent to the parasite FV. DNA, Hoechst 33342 (nuclei). Scale bars: 5 µm. (B-F) Immuno-transmission electron microscopy (TEM) using monoclonal mouse anti-PRDX6 antibody. (B) Uninfected RBC showing even cytosolic staining for PRDX6. (C-F) *P. falciparum-infected* RBCs show co-localization of PRDX6 and hemoglobin in vesicles at the inner surface of the parasite plasma membrane in structures that resemble cytostomes (black arrows), at the FV (white arrows), or within the parasite cytosol (striped grey arrow). Scale bars: 500 nm. Representative images from three independent experiments are shown.

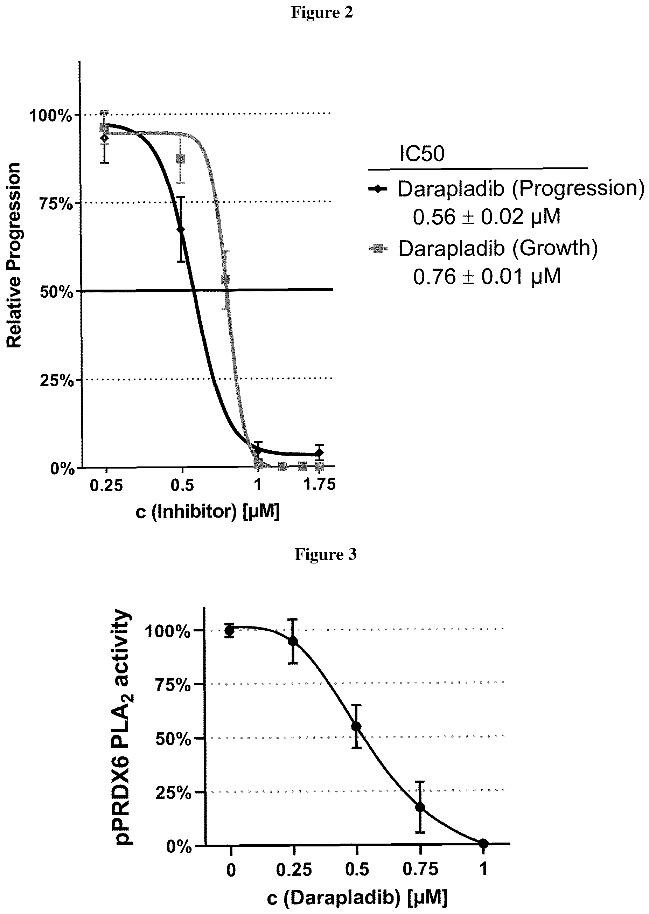

**Figure 2** shows inhibition of *P.falciparum* (3D7 strain) ring to schizont progression and blood stage growth by Darapladib. Parasites were treated at ring stage and growth to schizont stage in the same cycle ("progression") or to next generation rings ("growth") was scored by flow cytometry using DNA intercalating fluorescent dye SYBR Green I.
**Figure 3** concerns the radioactive PLA₂ activity assay using phosphorylated recombinant human PRDX6 in presence or absence of Darapladib. Darapladib inhibits PLA₂ activity of PRDX6 with IC50 of ≈0.5µM.

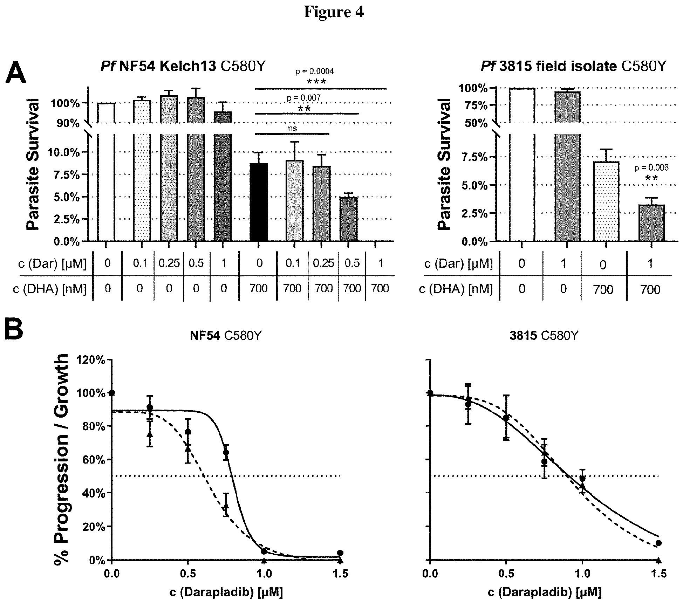

**Figure 4** is related to the co-treatment of ART-resistant parasites with artemisinin and Darapladib synergistically reduces parasite survival. (A) Ring stage survival assays (RSA) (Witkowski et al., Lancet Infect. Dis. 13, 1043-1049 (2013)) with Darapladib (Dar) and Dihydroartemisinin (DHA) against ART-resistant *P. falciparum* strains. For detailed data regarding synergism, cf. **Table 1.** (**B**) Growth/Progression assay with continuous Darapladib treatment against ART-resistant *P. falciparum* strains. Data are means ± SD of three independent experiments, unpaired t-tests. Darapladib thus synergistically restores susceptibility in Artemisinin-resistant strains in co-treatment with Artemisinin.

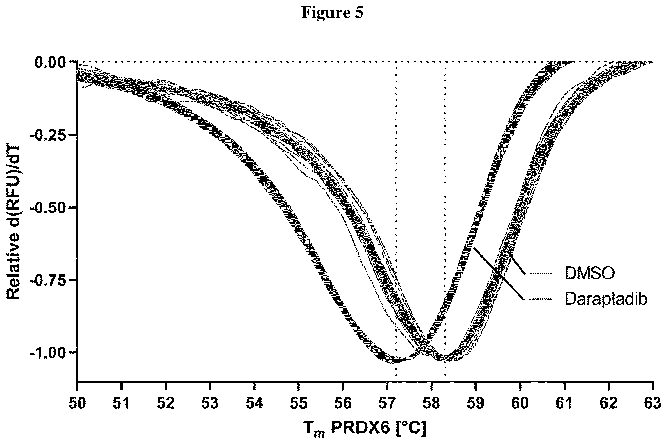

**Figure 5** shows that Darapladib induces a significant thermal shift in PRDX6. The thermal shift of the melting temperature of PRDX6 in presence of Darapladib was determined using a SYPRO orange-based differential scanning fluorimetry (DSF) assay. ΔTₘ > 0.5°C was considered significant. Incubation with 25µM Darapladib decreased the Tₘ of PRDX6 by 1.1 ± 0.2 °C.

### EXAMPLES

### Example 1: Synthesis of compounds of the invention

Darapladib and its derivatives, in particular compounds of formula (IV), with R_{c} of formula (A) and Rd of formula (B), are obtained according to the procedures described in patent applications EP 1 686 119 and EP 1 263 740.

### Example 2 : Compounds of the invention inhibit PRDX6 and blocks blood stage growth by impairment of lipid oxidation repair.

Darapladib has been assessed for the binding to the PLA₂ active site of PRDX6 by activity-based protein profiling (ABPP) using recombinant human PRDX6 and the fluorescently tagged probe TAMRA-FP that covalently labels the serine in the PLA₂ active site of PRDX6.

Darapladib bound the PLA2 active site of PRDX6, competed with TAMRA-FP and reduced the fluorescent labelling of PRDX6.

Darapladib was also tested *in vitro* for its ability to block *P. falciparum* blood stage growth and progression from rings to schizonts. Synchronous *P. falciparum* 3D7 cultures were treated at the ring stage and effects on ring to schizont development ("progression") and multiplication following completion of a full blood stage cycle ("growth") were assessed by flow cytometry using the DNA intercalating fluorescent dye SYBR Green I.

Darapladib blocked parasite progression and growth, and arrested parasite development at the trophozoite stage (Figure 2).

The IC₅₀ values for inhibition of progression (ring stage to schizont) and growth (whole cycle, ring to ring) are as follows (for all n = 3 in triplicates): Darapladib displayed an IC₅₀ for parasite progression (0.56 µM) and growth (0.76 µM) (Figure 2). Darapladib is thus an effective inhibitor of the blood stage of *P. falciparum.*

Darapladib also inhibited the PLA2 activity of PRDX6 in a PLA2 activity assay (Figure 3).

The specificity of Darapladib for PRDX6 was furthermore validated using ITDR-CETSA (IsoThermal Dose Response-Cellular Thermal Shift Assay), which demonstrated that no other essential host or parasite protein is targeted by Darapladib.

### Example 3 : Co-treatment of artemisinin-resistant parasites with artemisinin and Darapladib synergistically reduces parasite survival

It has been investigated whether co-treatment with Darapladib and ART could restore ART-susceptibility in the ring stage survival assay (RSA).

Co-treatment of of *P. falciparum* ring stages with Darapladib and Dihydroartemisinin (DHA), one of the ART derivatives used as first-line antimalarials, synergistically reduced survival of two artemisinin-resistant *P. falciparum* strains, namely, the genetically engineered clone NF54 Kelch13^{C580Y} and the 3815 strain, isolated from a Cambodian patient in a ring stage survival assay (RSA) (Figure 4).

Darapladib effectively blocked blood stage growth of both strains following continuous treatment during the complete blood stage cycle (Figure 4).

Co-treatment with Darapladib and Artemisinin thus synergistically reduces parasite survival in ring stage survival assay (RSA). This is further illustrated in following table 1 (the data corresponds to Figure 4).

The survival rate for treatment with 1 µM Darapladib alone and 700 nM DHA alone was multiplied to calculate a theoretical survival rate if the effect of the combination was additive ("Calc. Additive"). The experimentally measured survival rate ("Measured") was divided by the calculated theoretical additive survival rate for the co-treatment. A ratio < 1 for Measured/Calc. indicates synergism, a ratio = 1 indicates an additive effect and a ratio > 1 indicates an antagonistic effect. Data are means ± SD of six independent experiments for NF54 C580Y and three independent experiments for 3815, unpaired t-test.

**Table 1**

| | **Parasite survival** | | | | |
|---|---|---|---|---|---|
| | **Drugs Alone** | | **Combination** | | **Ratio** |
| **Strain** | **Dar 1µM** | **DHA** | **Calc. Additive** | **Measured** | **Measur./Calc.** |
| NF54 C580Y | 89.0 ± 8.1% | 7.9 ± 1.7% | 7.0 ± 2.1% | 0.1 ± 0.3% | 0.01 ^{∗∗∗∗} (p<0.0001) |
| 3815 | 94.7 ± 3.2% | 7.1 ± 0.9% | 6.7 ± 1.1% | 3.3 ± 0.5% | 0.49 ^{∗∗} (p=0.008) |

Darapladib could thus both reduce survival of ART-resistant parasites when combined with artemisinin as well as independently block parasite growth of ART-resistant *P. falciparum* strains. Co-treatment with ART and Darapladib synergistically reduced survival of ART-resistant parasites. Compounds of the invention thus play a pivotal role in restoring the efficacy of artemisinin against ART-resistant strains, by synergistically reducing ART-resistant parasite survival during early ring stage, restoring ART-susceptibility, and by independently inhibiting blood stage growth of *P. falciparum* isolates including ART-resistant strains.

### Example 4 : Biological evaluation of compounds of the invention

A differential scanning fluorimetry (DSF) HTS assay for PRDX6 has been established according to Bergsdorf et al., Methods Enzymol. 610, 135-165 (2018).

Binding of the compounds of the invention to PRDX6 induces a significant shift in the melting temperature of PRDX6 which can be detected using the hydrophobic fluorescent dye SYPRO orange (Figure 5). Compounds of the invention are identified by the respective shift in the melting temperature of PRDX6 induced by their binding.

Compounds of the invention are further tested at different concentrations for inhibition of *P. falciparum* blood stage growth *in vitro* using an established and regularly performed SYBR Green based flow cytometric assay to determine their IC₅₀ for blocking of in vitro blood stage growth of malaria parasites (Figure 2) (Izumiyama et al., Exp. Parasitol. 121, 144-150 (2009)).

To assess the compounds of the invention in an *in vivo* system, Wildtype B6J mice are infected with GFP-expressing *P. berghei* parasites and treated with different doses of identified hits. Growth of *P. berghei-GFP* are monitored daily by flow cytometry to determine IC₅₀ for *in vivo* parasite killing (Ishino et al., Mol. Microbiol. 59, 1175-1184 (2006)).

The enzymatic inhibition of PRDX6 by compounds of the invention hits are further examined using an established PLA2 activity assay using [¹⁴C]-labelled phospholipids (Figure 3) (Fisher et al., Am. J. Physiol. - Lung Cell. Mol. Physiol. 267, L335--341 (1994)).

## Claims

1. A compound of following formula (I): 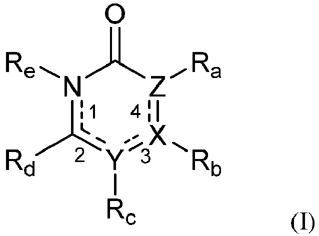 wherein:
- X is C, Y is C and Z is N, bonds 1 and 3 being double bonds, bonds 2 and 4 being single bonds; or
- X is C, Y is N and Z is C, bond 3 is a single bond and bond 4 is a double bond, and:
∘ bond 1 is a double bond, bond 2 is a single bond, Rₑ being absent; or
∘ bond 1 is a singe bond, bond 2 is a double bond, R_{c} being absent; or
- X is N, Y is C, Z is C, bonds 1 and 3 being single bonds, bonds 2 and 4 being double bonds; or
- X and Z are C and Y-R_{c} is 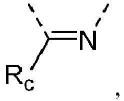 bonds 1, 2 and 3 are single bonds, bond 4 is a double bond;
when Z is C, Rₐ is hydrogen, halogen, (C₁-C₃)-alkyl, (C₁-C₃)-alkoxy, hydroxyl-(C₁-C₃)-alkyl, (C₁-C3)-alkylthio, (C₁-C₃)-alkylsulphinyl, amino-(C₁-C₃)-alkyl, mono- or di-(C₁-C₃)-alkylamino-(C₁-C3)-alkyl, (C₁-C₃)-alkylcarbonylamino-(C₁-C₃)-alkyl, (C₁-C₃)-alkoxy-(C₁-C₃)-alkylcarbonylamino-(C₁-C₃)-alkyl, (C₁-C₃)-alkylsulphonylamino-(C₁-C₃)-alkyl, (C₁-C₃)-alkylcarboxy, (C₁-C₃)-alkylcarboxy(C₁-C₃)-alkyl, aryl, in particular phenyl, or aralkyl, in particular benzyl, said aryl being optionally substituted by 1, 2, 3 or 4 substituents which may be the same or different selected from halogen, (C₁-C₄)-alkyl, CN, CF3, OCF3, COR2, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio, or mono to perfluoro-(C₁-C₄)-alkyl);
when Z is N, Rₐ is hydrogen, NR₄R₅, (C₁-C₆)-alkyl, (C₁-C₄)-alkoxy-(C₁-C₆)-alkyl, aryl, aryl-(C₁-C6)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C6)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
R_{b} is hydrogen, halogen, (C₁-C₃)-alkyl, hydroxyl-(C₁-C₃)-alkyl, NR₄R₅, in particular NH₂, OH or (C₁-C₃)-alkoxy, with the proviso that Rₐ and R_{b} are not simultaneously each hydrogen; or
when Y is N, Rₐ and R_{b} together may be (CH₂)ₙ where n is 3 or 4, to form, with the main ring carbon atoms to which they are attached a fused 5-or 6-membered carbocyclic ring; or
when Y is N, Rₐ and R_{b} together with the main ring carbon atoms to which they are attached may form a fused benzo or heteroaryl ring optionally substituted by 1, 2, 3 or 4 substituents which may be the same or different selected from halogen, (C₁-C₄)-alkyl, CN, CF3, OCF3, COR2, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio, or mono to perfluoro-(C₁-C₄)-alkyl); or
when Y is N, Rₐ and R_{b} together with the main ring carbon atoms to which they are attached may form a fused heterocycle, in particular a fused azaheterocycle, optionally substituted by 1, 2, 3 or 4 substituents which may be the same or different selected from (C₁-C₄)-alkyl, oxo, aryl or heteroaryl ring which is further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy), (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
said fused azaheterocycle optionally comprises at least one N-R_{a'} member;
R_{a'} is (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
R₂ and R₃ are independently hydrogen or (C₁-C₂₀)-alkyl, for instance (C₁-C₄)-alkyl;
R_{2'} is R₂ or NR₄R₅;
R3_{'} is (C₁-C₁₂)-alkyl, preferably (C₁-C₆)-alkyl, an aryl or heteroaryl group, said group being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl;
R₄ is hydrogen, (C₁-C₁₂)-alkyl, preferably (C₁-C₆)-alkyl, (C₁-C₁₂)-alkyl-R_{4'}, preferably (C₁-C₆)-alkyl-R_{4'};
R_{4'} is NH₂, mono- or di-(C₁-C₃)-alkylamino;
R₅ is hydrogen, (C₁-C₁₂)-alkyl, preferably (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl group being optionally fused with a cycloalkane, in particular a (C₅-C₈)-cycloalcane or a 5- to 8- membered heterocycle comprising N and optionally O or S, and being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl; or
R₄ and R₅ together with the nitrogen to which they are attached form a 5- to 8 membered ring optionally containing one or more further heteroatoms selected from oxygen, nitrogen, notably as N-R_{a'} member, and sulphur, optionnaly fused with an aromatic ring, preferably a benzene ring, said rings being optionally substituted by one or two substituents selected from hydroxy, oxo, (C₁-C4)-alkyl, (C₁-C₄)-alkyl-CO, aryl, in particular phenyl, or aralkyl, in particular benzyl, for example morpholine or piperazine; or
R₄ and R₅ which may be the same or different is each selected from CH₂R₆, CHR₇CO₂H or a salt thereof in which:
R₆ is COOH or a salt thereof, COOR₈, CONR₂R₃, CN, CH₂OH or CH₂OR₂;
R₇ is an amino acid side chain such as CH₂OH from serine;
R₈ is (C₁-C₄)-alkyl or a pharmaceutically acceptable *in vivo* hydrolysable ester group;
When Y is N, R_{c} is (C₁-C₆)-alkyl, aryl, aryl-(C₁-C₆)-alkyl, in particular an aryl-(C₁-C₂)-alkyl,
heteroaryl or heteroaryl-(C₁-C₆)-alkyl, in particular a heteroaryl-(C₁-C₂)-alkyl, said aryl or
heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy; or
When Y is N, R_{c} is of following formula (A): 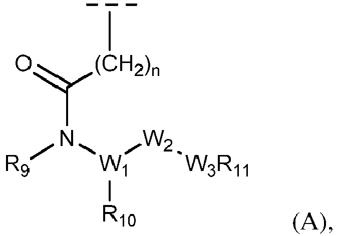
R₉ is hydrogen, (C₁-C₆)-alkyl which may be unsubstituted or substituted by 1, 2 or 3 substituents selected from hydroxy, halogen, OR2, COR2, carboxy, COOR2, CONR₄R₅, NR₄R₅, NR₄COR₅, mono-or di-(hydroxyl-(C₁-C₆)-alkyl)amino and N-hydroxy-(C₁-C₆)-alkyl-N-(C₁-C₆)-alkylamino, for example 1-piperidinoethyl; or
R₉ is Het-(C₀-C₄)-alkyl in which Het is a 5- to 7- membered heterocyclyl ring comprising N and optionally O or S, bonded through a carbon ring atom and in which N may be substituted by COR₂, COOR2, CONR₄R₅, or (C₁-C₆)-alkyl optionally substituted by 1, 2 or 3 substituents selected from hydroxy, halogen, OR2, COR2, carboxy, COOR2, CONR₄R₅, NR₄R₅, NR₄COR₅, for instance, piperidin-4-yl, pyrrolidin-3-yl;
R₁₀ is hydrogen or (C₁-C₃)-alkyl;
R₁₁ is an aryl or heteroaryl ring which is further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy), (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
W₁ is (CH₂)ₚ(O)_{q} in which p is 1, 2 or 3 and q is 0 or p is 2 or 3 and q is 1;
W2 is an arene diyl or heteroarene diyl group which is further optionally substituted by 1, 2, 3 or 4 substituents which may be the same or different selected from (C₁-C₁₈)-alkyl, in particular (C₁-C6)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl and mono to perfluoro-(C₁-C₄)-alkoxy;
W₃ is O or a bond;
when Y is C, R_{c} is H or in particular (C₁-C₆)-alkyl; or
when X and Y are C, R_{c} and R_{b} together may be (CH₂)ₙ where n is 3 or 4, to form, with the main ring carbon atoms to which they are attached a fused 5-or 6-membered carbocyclic ring;
when Y-R_{c} is 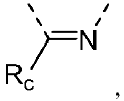 R_{c} is OH or (C₁-C₁₈)-alkoxy, in particular (C₁-C₆)-alkoxy;
R_{d} is H or (C₁-C₆)-alkyl; or
Rd is of the following formula (B): 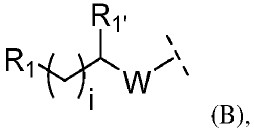
i is 0 or 1;
W is S, O, or CH2, in particular S;
R₁ is COOR2, COR_{2'}, OR_{3'}, an aryl or heteroaryl group, said group being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C4)-alkyl, mono to perfluoro-(C₁-C₄)-alkoxyaryl, and aryl-(C₁-C₄)-alkyl;
R_{1'} is H or (C₁-C₃)-alkyl;
when X is N, R_{c} and R_{d} may together with the main ring carbon atoms to which they are attached form a fused benzo or heteroaryl ring optionally substituted by 1, 2, 3 or 4 substituents which may be the same or different selected from halogen, (C₁-C₄)-alkyl, CN, CF3, OCF3, COR2, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio, or mono to perfluoro-(C₁-C₄)-alkyl);
Rₑ is H, (C₁-C₆)-alkyl, (C₁-C₆)-alkyl-R_{e'}, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
Re_{'} is COR2, carboxy, COOR2, CONR4R5;
with the proviso that:
- R_{d} is of formula (B); and/or
- Y is N, and R_{c} is aryl, aryl-(C₁-C₆)-alkyl, heteroaryl, heteroaryl-(C₁-C₆)-alkyl, or of formula (A); and/or
- Rₑ is (C₁-C₆)-alkyl-R_{e'}, aryl, aryl-(C₁-C₆)-alkyl, heteroaryl or heteroaryl-(C₁-C₆)-alkyl;
or a pharmaceutically acceptable salt or solvate thereof,
for use in the treatment and/or prevention of malaria.

2. The compound for use according to claim 1, of following formula (II): 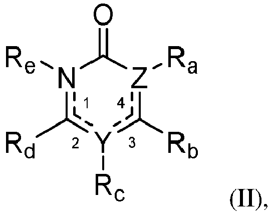 in particular of following formula (IIa): 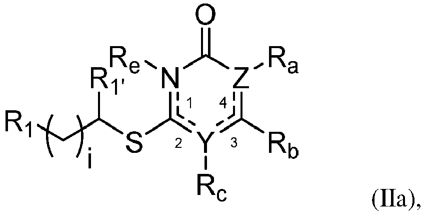 Wherein :
- Y is C and Z is N, bonds 1 and 3 being double bonds, bonds 2 and 4 being single bonds; or
- Y is N and Z is C, bond 3 is a single bond and bond 4 is a double bond, and:
∘ bond 1 is a double bond, bond 2 is a single bond, Rₑ being absent; or
∘ bond 1 is a singe bond, bond 2 is a double bond, R_{c} being absent.

3. The compound for use according to claim 1, of following formula (III): 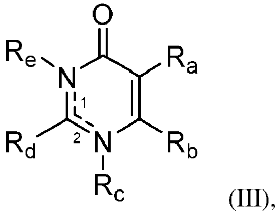 in particular of following formula (IIIa): 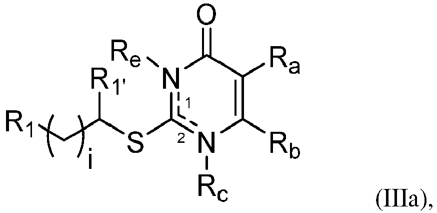 Wherein :
bond 1 and bond 2 are a single or a double bond, with:
- bond 1 is a double bond, bond 2 is a single bond, Rₑ being absent; or
- bond 1 is a singe bond, bond 2 is a double bond, R_{c} being absent.

4. The compound for use according to claim 1, of following formula (IV): 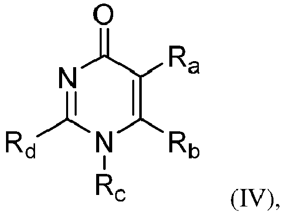 Wherein Rₐ, R_{b}, R_{c} and R_{d} are as defined in claim 1, and in particular wherein:
Rₐ is aryl, in particular phenyl, or aralkyl, in particular benzyl, said aryl being optionally substituted by 1, 2, 3 or 4 substituents which may be the same or different selected from halogen, (C₁-C₄)-alkyl, CN, CF3, OCF3, COR2, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio, or mono to perfluoro-(C₁-C₄)-alkyl), said aryl being more particularly phenyl;
R_{b} is hydrogen or (C₁-C₃)-alkyl, in particular hydrogen ;
said fused azaheterocycle optionally comprises at least one N-R_{a'} member;
R_{a'} is (C₁-C₆)-alkyl, aryl, aryl-(C₁-C₆)-alkyl, in particular an aryl-(C₁-C₂)-alkyl, heteroaryl or
heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
R₂ and R₃ are independently hydrogen or (C₁-C₂₀)-alkyl, for instance (C₁-C₄)-alkyl;
R_{2'} is R₂ or NR₄R₅;
R_{3'} is (C₁-C₁₂)-alkyl, preferably (C₁-C₆)-alkyl, an aryl or heteroaryl group, said group being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl;
R₄ is hydrogen, (C₁-C₁₂)-alkyl, preferably (C₁-C₆)-alkyl, (C₁-C₁₂)-alkyl-R_{4'}, preferably (C₁-C₆)-alkyl-R_{4'};
R_{4'} is NH₂, mono- or di-(C₁-C₃)-alkylamino;
R₅ is hydrogen, (C₁-C₁₂)-alkyl, preferably (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl,
said aryl or heteroaryl group being optionally fused with a cycloalkane, in particular a (C₅-C₈)-cycloalcane or a 5- to 8- membered heterocycle comprising N and optionally O or S, and being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl; or
R₄ and R₅ together with the nitrogen to which they are attached form a 5- to 8 membered ring optionally containing one or more further heteroatoms selected from oxygen, nitrogen, notably as N-R_{a'} member, and sulphur, optionnaly fused with an aromatic ring, preferably a benzene ring, said rings being optionally substituted by one or two substituents selected from hydroxy, oxo, (C₁-C₄)-alkyl, (C₁-C₄)-alkyl-CO, aryl, in particular phenyl, or aralkyl, in particular benzyl, for example morpholine or piperazine;
R_{c} is aryl-(C₁-C₆)-alkyl, in particular an aryl-(C₁-C₂)-alkyl, or heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy, more particularly from (C₁-C₆)-alkyl, aryl-(C₁-C₆)-alkoxy and OCF₃;
R_{d} is H or (C₁-C₆)-alkyl.

5. The compound for use according to claim 1, of following formula (IVa): 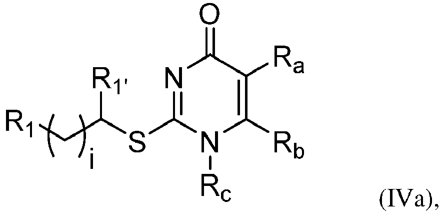 Wherein Rₐ, R_{b}, R_{c}, R₁, R_{1'} and i are as defined in claim 1, and in particular wherein:
Rₐ is hydrogen, aryl, in particular phenyl, or aralkyl, in particular benzyl, more particularly hydrogen or benzyl;
R_{b} is NR₄R₅, in particular NH₂, or OH; or
Rₐ and R_{b} together with the main ring carbon atoms to which they are attached may form a fused benzo or heteroaryl ring optionally substituted by 1, 2, 3 or 4 substituents which may be the same or different selected from halogen, (C₁-C₄)-alkyl, CN, CF3, OCF3, COR2, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio, or mono to perfluoro-(C₁-C₄)-alkyl), in particular from (C₁-C₄)-alkyl and COOR2; or
Rₐ and R_{b} together with the main ring carbon atoms to which they are attached may form a fused heterocycle, in particular a fused azaheterocycle, optionally substituted by 1, 2, 3 or 4 substituents which may be the same or different selected from (C₁-C₄)-alkyl, oxo, aryl or heteroaryl ring which is further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy), (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy, more particularly from oxo, aryl and halogen;
said fused azaheterocycle optionally comprises at least one N-R_{a'} member;
R_{a'} is (C₁-C₆)-alkyl, aryl, aryl-(C₁-C₆)-alkyl, in particular an aryl-(C₁-C₂)-alkyl, said aryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
R₂ and R₃ are independently hydrogen or (C₁-C₂₀)-alkyl, for instance (C₁-C₄)-alkyl;
R_{2'} is R₂ or NR₄R₅;
R₄ is hydrogen, (C₁-C₁₂)-alkyl, preferably (C₁-C₆)-alkyl;
R₅ is hydrogen, (C₁-C₁₂)-alkyl, preferably (C₁-C₆)-alkyl, aryl, heteroaryl or said aryl or heteroaryl group being optionally fused with a cycloalkane, in particular a (C₅-C₈)-cycloalcane and being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl, more particularly from halogen, CN, COOR2; or
R₄ and R₅ together with the nitrogen to which they are attached form a 5- to 8 membered ring optionally containing one or more further heteroatoms selected from oxygen, nitrogen, notably as N-R_{a'} member, and sulphur, optionnaly fused with an aromatic ring, preferably a benzene ring, said rings being optionally substituted by one or two substituents selected from hydroxy, oxo, (C₁-C4)-alkyl, (C₁-C₄)-alkyl-CO, aryl, in particular phenyl, or aralkyl, in particular benzyl, for example morpholine or piperazine; or
R_{c} is (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, more particularly (C₁-C₆)-alkyl, aryl, or aryl-(C₁-C₆)-alkyl, said aryl or heteroaryl being further optionally substituted by 1, 2, 3 or 4 substituents which may be the same or different selected from (C₁-C₁₈)-alkyl, in particular (C₁-C6)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy, more particularly from halogen, (C₁-C₆)-alkoxy and CF3;
i is 0 or 1, in particular 0;
W is S, O, or CH2, in particular S;
R₁ is COOR₂, COR_{2'}, OR_{3'}, an aryl or heteroaryl group, said group being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C4)-alkyl, mono to perfluoro-(C₁-C₄)-alkoxyaryl, and aryl-(C₁-C₄)-alkyl, more particularly halogen;
R_{1'} is H or (C₁-C₃)-alkyl, in particular H;
R_{3'} is an aryl or heteroaryl group, in particular an aryl, said group being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C4)-alkyl, mono to perfluoro-(C₁-C₄)-alkoxyaryl, and aryl-(C₁-C₄)-alkyl, more particularly being CF₃.

6. The compound for use according to claim 1, of following formula (V): 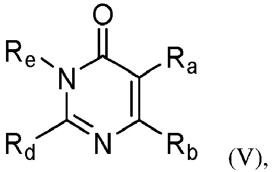 Wherein Rₐ, R_{b}, R_{d}, and Rₑ, are as defined in claim 1, and in particular wherein:
Rₐ and R_{b} together with the main ring carbon atoms to which they are attached form a fused benzo or heteroaryl ring optionally substituted by 1, 2, 3 or 4 substituents which may be the same or
different selected from halogen, (C₁-C₄)-alkyl, CN, CF3, OCF3, COR2, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio, or mono to perfluoro-(C₁-C₄)-alkyl);
R_{a'} is (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
R₂ and R₃ are independently hydrogen or (C₁-C₂₀)-alkyl, for instance (C₁-C₄)-alkyl;
R_{2'} is R₂ or NR₄R₅;
R₄ is hydrogen, (C₁-C₁₂)-alkyl, preferably (C₁-C₆)-alkyl, (C₁-C₁₂)-alkyl-R_{4'}, preferably (C₁-C₆)-alkyl-R_{4'};
R4 being in particular (C₁-C₆)-alkyl-R_{4'};
R_{4'} is NH₂, mono- or di-(C₁-C₃)-alkylamino, in particular di-(C₁-C₃)-alkylamino;
R₅ is hydrogen, (C₁-C₁₂)-alkyl, preferably (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl group being optionally fused with a cycloalkane, in particular a (C₅-C₈)-cycloalcane or a 5- to 8- membered heterocycle comprising N and optionally O or S, and being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl, more particularly halogen; or
R₅ being in particular arly or substituted aryl; or
R₄ and R₅ together with the nitrogen to which they are attached form a 5- to 8 membered ring optionally containing one or more further heteroatoms selected from oxygen, nitrogen, notably as N-R_{a'} member, and sulphur, optionnaly fused with an aromatic ring, preferably a benzene ring, said rings being optionally substituted by one or two substituents selected from hydroxy, oxo, (C₁-C4)-alkyl, (C₁-C₄)-alkyl-CO, aryl, in particular phenyl, or aralkyl, in particular benzyl, for example morpholine or piperazine;
Rd is H or (C₁-C₆)-alkyl, in particular H;
Rₑ is (C₁-C₆)-alkyl-R_{e'};
R_{e'} is COR2, carboxy, COOR2, CONR₄R₅, in particular CONR₄R₅.

7. The compound for use according to claim 1, of following formula (Va): 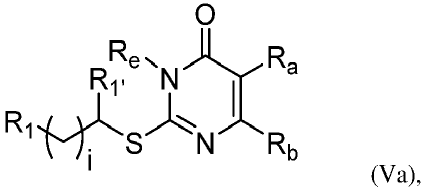 Wherein Rₐ, R_{b}, Rₑ, R₁, R_{1'} and i are as defined in claim 1, and in particular wherein:
Rₐ and R_{b} together with the main ring carbon atoms to which they are attached may form a fused benzo or heteroaryl ring optionally substituted by 1, 2, 3 or 4 substituents which may be the same or different selected from halogen, (C₁-C₄)-alkyl, CN, CF3, OCF3, COR2, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio, or mono to perfluoro-(C₁-C₄)-alkyl);
R₂ and R₃ are independently hydrogen or (C₁-C₂₀)-alkyl, for instance (C₁-C₄)-alkyl;
R_{2'} is R₂ or NR₄R₅;
R₄ is hydrogen;
R₅ is aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl group being optionally fused with a cycloalkane, in particular a (C₅-C₈)-cycloalcane or a 5- to 8- membered heterocycle comprising N and optionally O or S, and being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl, more particularly from (C₁-C₁₈)-alkyl; or
i is 0 or 1, in particular 0;
W is S, O, or CH2, in particular S;
R₁ is an aryl or heteroaryl group, in particular an aryl, said group being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl, more particularly from halogen;
R₁, is H or (C₁-C₃)-alkyl, in particular H;
Rₑ is (C₁-C₆)-alkyl-R_{e'};
R_{e'} is COR2, carboxy, COOR2, CONR₄R₅, in particular CONR₄R₅.

8. The compound for use according to claim 1, of following formula (VI): 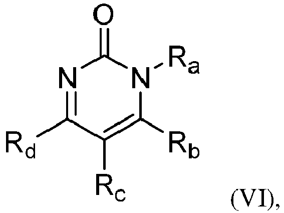 wherein Rₐ, R_{b}, R_{c}, and R_{d} are as defined in claim 1.

9. The compound for use according to claim 1, of following formula (VIa): 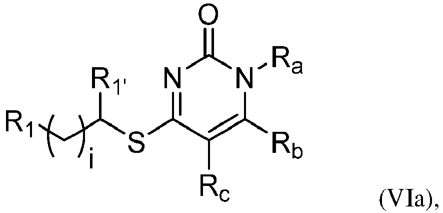 Wherein Rₐ, R_{b}, R_{c}, R₁, R_{1'} and i are as defined in claim 1, and in particular wherein:
Rₐ is NR₄R₅, (C₁-C₄)-alkoxy-(C₁-C₆)-alkyl, aryl-(C₁-C₆)-alkyl, in particular an aryl-(C₁-C₂)-alkyl, heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
R_{a'} is (C₁-C₆)-alkyl, aryl, aryl-(C₁-C₆)-alkyl, in particular an aryl-(C₁-C₂)-alkyl, heteroaryl or
heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy, R_{a'} being more particularly (C₁-C₆)-alkyl;
R₂ and R₃ are independently hydrogen or (C₁-C₂₀)-alkyl, for instance (C₁-C₄)-alkyl;
R_{2'} is R₂ or NR₄R₅;
R₄ is hydrogen, (C₁-C₁₂)-alkyl, preferably (C₁-C₆)-alkyl, (C₁-C₁₂)-alkyl-R_{4'}, preferably (C₁-C₆)-alkyl-R_{4'};
R_{4'} is NH₂, mono- or di-(C₁-C₃)-alkylamino;
R₅ is hydrogen, (C₁-C₁₂)-alkyl, preferably (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl group being optionally fused with a cycloalkane, in particular a (C₅-C₈)-cycloalcane or a 5- to 8- membered heterocycle comprising N and optionally O or S, and being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl; or
R₄ and R₅, preferably, together with the nitrogen to which they are attached form a 5- to 8 membered ring optionally containing one or more further heteroatoms selected from oxygen, nitrogen, notably as N-R_{a'} member, and sulphur, optionnaly fused with an aromatic ring, preferably a benzene ring, said rings being optionally substituted by one or two substituents selected from hydroxy, oxo, (C₁-C₄)-alkyl, (C₁-C₄)-alkyl-CO, aryl, in particular phenyl, or aralkyl, in particular benzyl, for example morpholine or piperazine; or
R_{c} and R_{b} together may be (CH₂)ₙ where n is 3 or 4, to form, with the main ring carbon atoms to which they are attached a fused 5-or 6-membered carbocyclic ring;
i is 0 or 1, in particular 0;
W is S, O, or CH2, in particular S;
R₁ is an aryl or heteroaryl group, in particular aryl, said group being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl, more particularly from halogen and CF₃;
R_{1'} is H or (C₁-C₃)-alkyl, in particular H.

10. The compound for use according to claim 1, of following formula (VII): 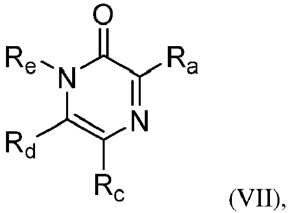 Wherein Rₐ, R_{c}, R_{d}, and Rₑ are as defined in claim 1, and in particular wherein:
Rₐ is hydrogen or (C₁-C₃)-alkyl, in particular hydrogen;
R_{a'} is (C₁-C₆)-alkyl, aryl, aryl-(C₁-C₆)-alkyl, in particular an aryl-(C₁-C₂)-alkyl, heteroaryl or
heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
R₂ and R₃ are independently hydrogen or (C₁-C₂₀)-alkyl, for instance (C₁-C₄)-alkyl;
R_{2'} is R₂ or NR₄R₅;
R₄ is hydrogen, (C₁-C₁₂)-alkyl, preferably (C₁-C₆)-alkyl, (C₁-C₁₂)-alkyl-R_{4'}, preferably (C₁-C₆)-alkyl-R_{4'};
R_{4'} is NH₂, mono- or di-(C₁-C₃)-alkylamino;
R₅ is hydrogen, (C₁-C₁₂)-alkyl, preferably (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl group being optionally fused with a cycloalkane, in particular a (C₅-C₈)-cycloalcane or a 5- to 8- membered heterocycle comprising N and optionally O or S, and being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl; or
preferably, R₄ and R₅ together with the nitrogen to which they are attached form a 5- to 8 membered ring optionally containing one or more further heteroatoms selected from oxygen, nitrogen, notably as N-R_{a'} member, and sulphur, optionnaly fused with an aromatic ring, preferably a benzene ring, said rings being optionally substituted by one or two substituents selected from hydroxy, oxo, (C₁-C₄)-alkyl, (C₁-C₄)-alkyl-CO, aryl, in particular phenyl, or aralkyl, in particular benzyl, for example morpholine or piperazine;
R_{c} and R_{d} may together with the main ring carbon atoms to which they are attached form a fused benzo or heteroaryl ring, in particular benzo, optionally substituted by 1, 2, 3 or 4 substituents which may be the same or different selected from halogen, (C₁-C₄)-alkyl, CN, CF₃, OCF₃, COR₂, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio, or mono to perfluoro-(C₁-C₄)-alkyl);
Rₑ is (C₁-C₆)-alkyl-R_{e'};
R_{e'} is COR2, carboxy, COOR2, CONR₄R₅, in particular CONR₄R₅.

11. The compound for use according to claim 1, of following formula (VIII): 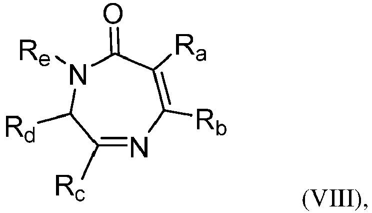 Wherein Rₐ, R_{b}, R_{c}, R_{d}, and Rₑ are as defined in claim 1, and in particular wherein:
Rₐ and R_{b} together with the main ring carbon atoms to which they are attached may form a fused benzo or heteroaryl ring, in particular benzo, optionally substituted by 1, 2, 3 or 4 substituents which may be the same or different selected from halogen, (C₁-C₄)-alkyl, CN, CF₃, OCF₃, COR₂, carboxy, COOR2, CONR₄R₅, NR₄R₅, SO₂NR₄R₅, NR₂SO₂R₃, NR₄R₅, (C₁-C₄)-alkoxy or (C₁-C₄)-alkylthio, or mono to perfluoro-(C₁-C₄)-alkyl);
said fused azaheterocycle optionally comprises at least one N-R_{a'} member;
R_{a'} is (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl being further optionally substituted by 1, 2, 3 or 4 substituents which may be the same or different selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxy, halogen, CN, CF3, OCF3, COR2, carboxy, COOR2, CONR₄R₅, NR₄R₅, SO₂NR₄R₅, NR₂SO₂R₃, NR₄R₅, mono to perfluoro-(C₁-C₄)-alkyl and mono to perfluoro-(C₁-C₄)-alkoxy;
R₂ and R₃ are independently hydrogen or (C₁-C₂₀)-alkyl, for instance (C₁-C₄)-alkyl;
R_{2'} is R₂ or NR₄R₅;
R₄ is hydrogen, (C₁-C₁₂)-alkyl, preferably (C₁-C₆)-alkyl, (C₁-C₁₂)-alkyl-R_{4'}, preferably (C₁-C₆)-alkyl-R_{4'};
R_{4'} is NH₂, mono- or di-(C₁-C₃)-alkylamino;
R₅ is hydrogen, (C₁-C₁₂)-alkyl, preferably (C₁-C₆)-alkyl, aryl, aryl-(C₁-C₆)-alkyl, in particular an aryl-(C₁-C₂)-alkyl, heteroaryl or heteroaryl-(C₁-C₆)-alkyl, in particular a heteroaryl-(C₁-C₂)-alkyl, said aryl or heteroaryl group being optionally fused with a cycloalkane, in particular a (C₅-C₈)-cycloalcane or a 5- to 8- membered heterocycle comprising N and optionally O or S, and being optionally substituted by 1, 2, 3 or 4 groups independently selected from (C₁-C₁₈)-alkyl, in particular (C₁-C₆)-alkyl, (C₁-C₁₈)-alkoxy, in particular (C₁-C₆)-alkoxy, (C₁-C₁₈)-alkylthio, in particular (C₁-C₆)-alkylthio, aryl-(C₁-C₁₈)-alkoxy, in particular aryl-(C₁-C₆)-alkoxy, hydroxyl, halogen, CN, CF3, OCF3, COR2, carboxy, COOR2, NR2COR3, CONR₄R₅, SO₂NR₄R₅, NR₂SO₂R₃, NR₄R₅, mono to perfluoro-(C₁-C₄)-alkyl, mono to perfluoro-(C₁-C₄)-alkoxyaryl, and aryl-(C₁-C₄)-alkyl; or
preferably, R₄ and R₅ together with the nitrogen to which they are attached form a 5- to 8 membered ring optionally containing one or more further heteroatoms selected from oxygen, nitrogen, notably as N-R_{a'} member, and sulphur, optionnaly fused with an aromatic ring, preferably a benzene ring, said rings being optionally substituted by one or two substituents selected from hydroxy, oxo, (C₁-C₄)-alkyl, (C₁-C₄)-alkyl-CO, aryl, in particular phenyl, or aralkyl, in particular benzyl, for example morpholine or piperazine;
R_{c} is OH or (C₁-C₁₈)-alkoxy, in particular (C₁-C₆)-alkoxy, R_{c} being notably OH;
Rd is of the following formula (B): 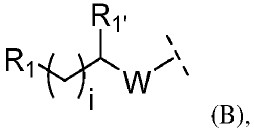
i is 0 or 1, in particular 0;
W is S, O, or CH2, in particular CH2;
R₁ is COOR₂ or COR_{2'},
R₁, is H or (C₁-C₃)-alkyl;
Rₑ is H or (C₁-C₆)-alkyl, in particular H.

12. The compound for use according to claim 1, of one of the following formulae:

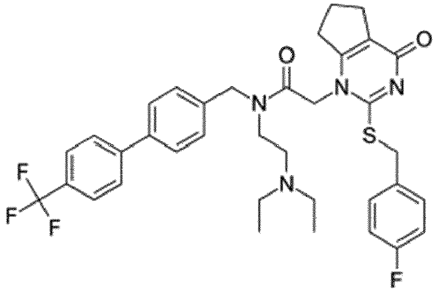

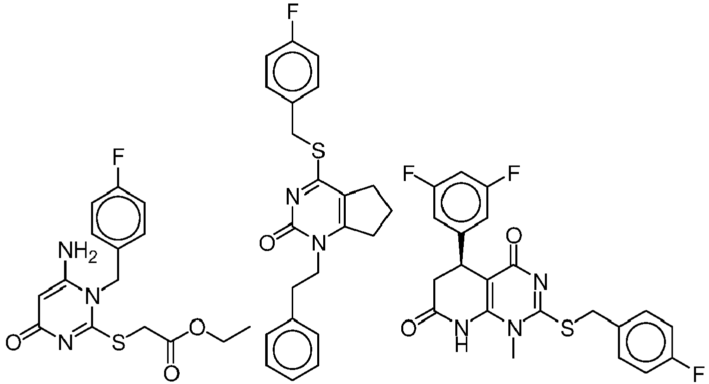

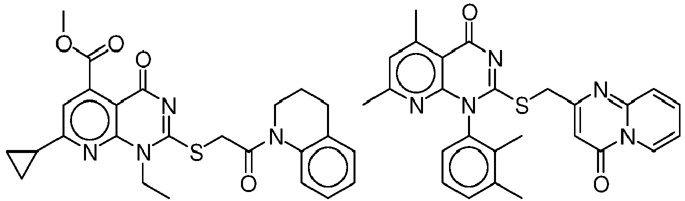

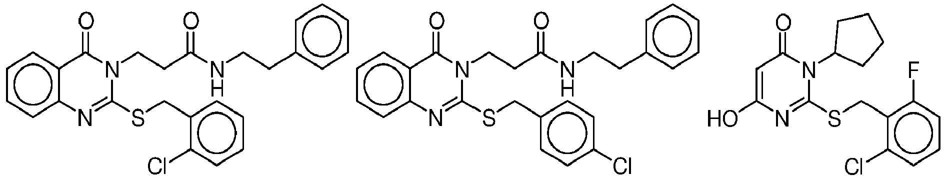

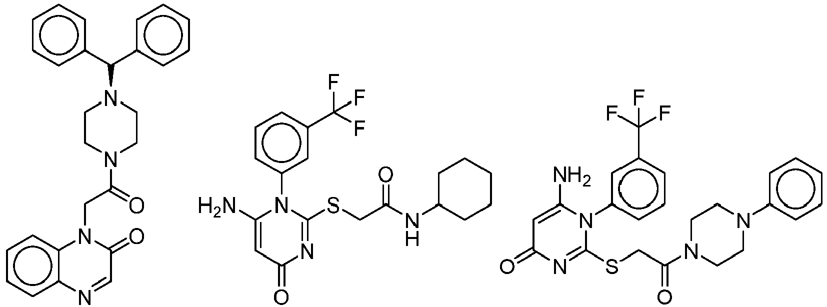

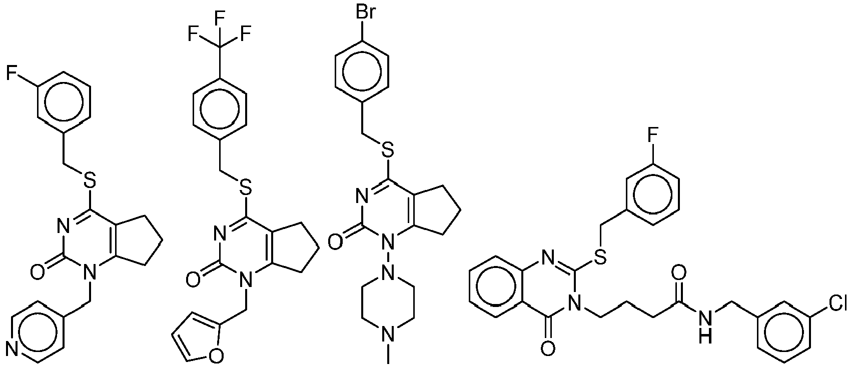

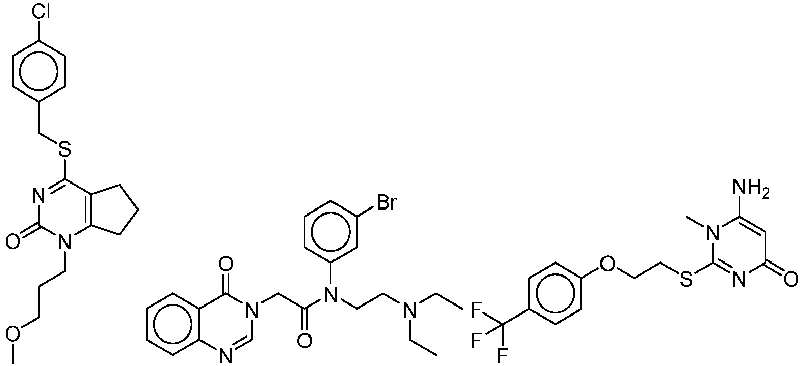

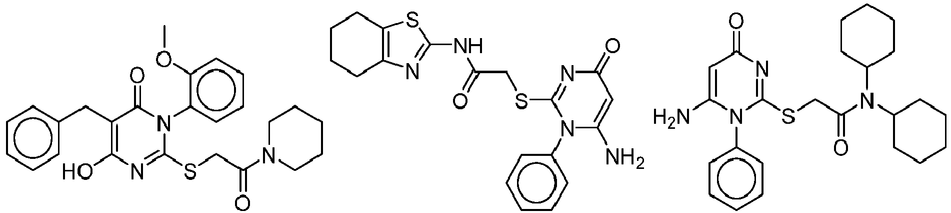

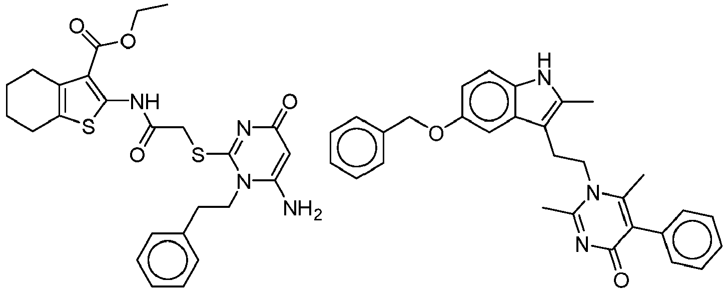

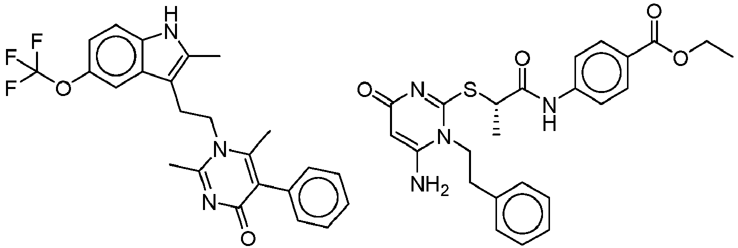

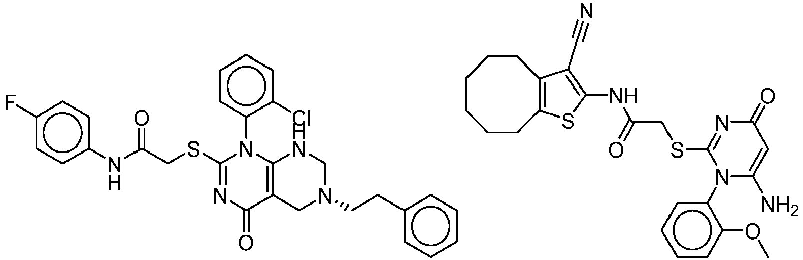

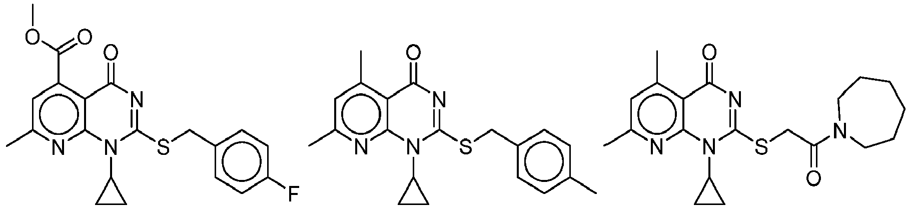

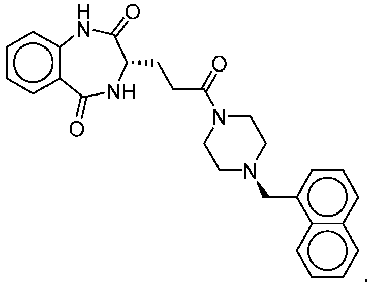

13. The compound for use according to any one of claims 1 to 12, wherein malaria is caused by a drug sensitive or drug resistant malaria parasite, in particular a drug resistant malaria parasite, more particularly an artemisinin-resistant malaria parasite.

14. The compound for use according to claim any one of claims 1 to 13, wherein malaria is caused by infection with Plasmodium, in particular by *Plasmodium falciparum, P. vivax, P. ovale,* or *P. malariae, P. knowlesi* more particularly by *Plasmodium falciparum.*

15. A combination of a compound according to any one of claims 1 to 12, with at least one other antimalarial agent for use in the treatment of malaria with simultaneous administration, separate or spread out over time, wherein the other antimalarial agent is in particular selected from chloroquine, artemesin, qinghaosu, 8-aminoquinoline, amodiaquine, arteether, artemether, artemisinin, artesunate, artesunic acid, artelinic acid, atovaquone, azithromycine, biguanide, chloroquine phosphate, chlorproguanil, cycloguanil, dapsone, desbutyl halofantrine, desipramine, doxycycline, dihydrofolate reductase inhibitors, dipyridamole, halofantrine, haloperidol, hydroxychloroquine sulfate, imipramine, mefloquine, penfluridol, phospholipid inhibitors, primaquine, proguanil, pyrimethamine, pyronaridine, quinine, quinidine, quinacrineartemisinin, sulfonamides, sulfones, sulfadoxine, sulfalene, tafenoquine, tetracycline, tetrandine, triazine, salts and mixture thereof, in particular artemisinin.
